(19) Europäisches Patentamt
European Patent Office
Office européen des brevets

(11) EP 1 192 122 B1

(12) FASCICULE DE BREVET EUROPEEN

(45) Date de publication et mention de la délivrance du brevet: **22.10.2003 Bulletin 2003/43**

(21) Numéro de dépôt: **00940462.5**

(22) Date de dépôt: **08.06.2000**

(51) Int Cl.[7]: **C07C 211/35**, A61K 31/135, A61P 37/00, A61P 35/00, A61P 9/00

(86) Numéro de dépôt international: **PCT/FR00/01575**

(87) Numéro de publication internationale: **WO 00/076953 (21.12.2000 Gazette 2000/51)**

(54) **DERIVES DU BENZENE, UN PROCEDE POUR LEUR PREPARATION ET LES COMPOSITIONS PHARMACEUTIQUES LES CONTENANT**

BENZOLDERIVATE, EIN VERFAHREN ZU IHRER HERSTELLUNG UND PHARMAZEUTISCHE ZUSAMMENSETZUNGEN, DIE DIESE ENTHALTEN

BENZENE DERIVATIVES, PREPARATION METHOD AND PHARMACEUTICAL COMPOSITIONS CONTAINING SAME

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **11.06.1999 FR 9907396**

(43) Date de publication de la demande: **03.04.2002 Bulletin 2002/14**

(73) Titulaire: **SANOFI-SYNTHELABO**
**75013 Paris (FR)**

(72) Inventeurs:
- **BOIGEGRAIN, Robert**
  **F-34820 Assas (FR)**
- **BOURRIE, Bernard**
  **F-34980 Saint Gely du Fesc (FR)**
- **BOURRIE, Martine**
  **F-34980 Saint Gely du Fesc (FR)**
- **CASELLAS, Pierre**
  **F-34090 Montpellier (FR)**
- **HERBERT, Jean, Marc**
  **F-31170 Tournefeuille (FR)**
- **LAIR, Pierre**
  **F-31120 Goyrans (FR)**
- **NISATO, Dino**
  **F-34680 Saint Georges d'Orques (FR)**
- **PAUL, Raymond**
  **F-34980 Saint Gely du Fesc (FR)**
- **VERNIERES, Jean, Claude**
  **F-31600 Muret (FR)**

(74) Mandataire: **Monain, Patrice et al**
**Sanofi-Synthelabo,**
**174 avenue de France**
**75013 Paris (FR)**

(56) Documents cités:
**EP-A- 0 376 850** **EP-A- 0 461 986**
**WO-A-98/04251**

- **BOURRIE B ET AL: "ENHANCEMENT OF ENDOTOXIN-INDUCED INTERLEUKIN-10 PRODUCTION BY SR 31747A, A SIGMA LIGAND" EUROPEAN JOURNAL OF IMMUNOLOGY,DE,WEINHEIM, vol. 25, no. 10, 1 octobre 1985 (1985-10-01), pages 2882-2887, XP000645468 ISSN: 0014-2980**
- **PAUL R ET AL: "ALLOSTERIC MODULATION OF PERIPHERAL SIGMA BINDING SITES BY A NEW SELECTIVE LIGAND: SR 31747" JOURNAL OF NEUROIMMUNOLOGY,XX,ELSEVIER SCIENCE PUBLISHERS BV, vol. 52, no. 2, 1 juillet 1994 (1994-07-01), pages 183-192, XP000601514 ISSN: 0165-5728**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 192 122 B1

- SILVE S ET AL: "THE IMMUNOSUPPRESSANT SR 31747 BLOCKS CELL PROLIFERATION BY INHIBITING A STEROID ISOMERASE IN SACCHAROMYCES CEREVISIAE" MOLECULAR AND CELLULAR BIOLOGY,US,WASHINGTON, DC, vol. 16, no. 6, 1 juin 1996 (1996-06-01), pages 2719-2727, XP000645472 ISSN: 0270-7306
- CASELLAS P ET AL: "IMMUNOPHARMACOLOGICAL PROFILE OF SR 31747: IN VITRO AND IN VIVO STUDIES ON HUMORAL AND CELLULAR RESPONSES" JOURNAL OF NEUROIMMUNOLOGY,XX,ELSEVIER SCIENCE PUBLISHERS BV, vol. 52, no. 2, 1 juillet 1994 (1994-07-01), pages 193-203, XP000645469 ISSN: 0165-5728

## Description

La présente invention concerne des dérivés du benzène comprenant une fonction amine substituée par un groupe alkyle et un groupe cycloalkyle, se liant spécifiquement aux récepteurs sigma, notamment à ceux du système nerveux périphérique, un procédé pour la préparation de ces composés et leur utilisation dans des compositions pharmaceutiques et plus particulièrement en tant qu'immunosupresseurs.

Les récepteurs sigma ont été mis en évidence à l'aide de plusieurs ligands. En premier lieu, on peut citer des composés opiacés, les 6,7-benzomorphans ou SKF-10,047, plus particulièrement le composé chiral (+) SKF-10,047 (W. R. Martin *et al.*, J. Pharmacol. Exp.Ther. 1976,*197*, 517-532 ; B. R. Martin *et al.*, J. Pharmacol. Exp. Ther. 1984, 231, 539-544). Parmi ces composés, les plus utilisés sont la (+) *N*-allylnormétazocine ou (+) NANM et la (+) pentazocine. Un neuroleptique, l'halopéridol, est également un ligand des récepteurs sigma, ainsi que le (+) 3-(3-hydroxyphényl)-1-propylpipéridine ou (+) 3-PPP (B. L. Largent *et al.*, Proc. Nat. Acad. Sci. USA 1984, 81, 4983-4987).

Le brevet US 4,709,094 décrit des dérivés de la guanidine très actifs comme ligands spécifiques des récepteurs sigma, plus particulièrement on peut citer la di-(*O*-tolyl) guanidine ou DTG. La distribution anatomique des récepteurs sigma dans le cerveau a été étudiée par autoradiographie, après marquage de ces récepteurs par la DTG selon E. Weber *et al.*, Proc. Nat. Acad. Sci. USA 1986, 83, 8784-8788, ainsi que par les ligands (+) SKF-10,047 et (+) 3-PPP selon B. L. Largent *et al.*, J. Pharmacol. Exp. Ther. USA 1986, 238, 739-748. L'étude par autoradiographie a permis d'identifier nettement les récepteurs sigma du cerveau et de les distinguer des autres récepteurs des opiacés, ainsi que ceux de la phencyclidine. Les récepteurs sigma sont particulièrement abondants dans le système nerveux central et concentrés dans le tronc cérébral, le système limbique et les régions impliquées dans la régulation des émotions. On trouve également des récepteurs sigma dans différents tissus périphériques. On distingue au moins deux types de récepteurs sigma : les récepteurs sigma-1 et les récepteurs sigma-2. Les ligands de type (+) SKF-10,047 se fixent sélectivement sur les récepteurs sigma-1 alors que d'autres ligands comme la DTG, l'halopéridol ou la (+) 3-PPP présentent une grande affinité à la fois pour les récepteurs sigma-1 et sigma-2.

Le brevet EP 0376850 décrit des composés de formule :

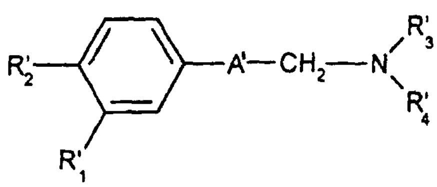

(A)

se liant sélectivement aux récepteurs sigma et possédant une activité immunosuppressive.

Parmi cette série de composés, le chlorhydrate de [3-(3-chloro-4-cyclohexylphényl) allyl]cyclohexyléthylamine, (Z) de formule :

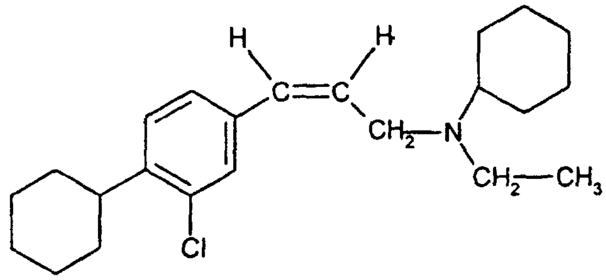

, HCl

a particulièrement été étudié. On pourra se référer par exemple à Biological Chemistry 1997, 272 (43), 27107-27115 ; Immunopharmacology and Immunotoxycology 1996, *18* (2), 179-191. Les composés de formule (A) ont cependant une propriété spécifique qui pourrait être considérée comme un inconvénient. Il s'agit d'une propriété apparaissant lors de la métabolisation : la dépendance au cytochrome nommé CYP 2D6.

En 1957, pour la première fois, on a envisagé que des différences héréditaires pouvaient être responsables des variations de réponse face aux médicaments.

Le métabolisme oxydatif montre des variations importantes selon les individus et les races. Les recherches effectuées ces 15 dernières années ont montré que les variations de l'expression fonctionnelle de la famille du cytochrome P450 (CYP) multigénique est à l'origine de ces différences. Seulement quelques isoformes du cytochrome P450 parmi celles déjà caractérisées chez l'homme ont un rôle dans le métabolisme oxydatif des médicaments. On pourra se référer à Xenobiotica, 1986, *16*, 367-378. Jusqu'à présent les CYP 1A2, CYP 2A6, CYP 2C9, CYP 2D6, CYP 2C19, CYP 2E1 et CYP 3A4 ont été identifiés pour leur importance clinique. Actuellement, on estime que les CYP 3A4, CYP 2D6 et CYP 2C9 sont responsables à eux seuls (et à des degrés variables) de 90% du métabolisme d'oxydation des médicaments. Bien que l'expression fonctionnelle de ces isoformes soit régulée et influencée par un bon nombre de facteurs environnementaux, physiologiques, les facteurs génétiques ont l'influence la plus marquée, ce qui souligne le rôle important que joue le polymorphisme dans l'oxydation des médicaments. Un certain nombre de ces polymorphismes ont été étudiés (particulièrement ceux du CYP 2C19 et du CYP 2D6).

Plus particulièrement, l'importance clinique du polymorphisme du CYP 2D6 dans la 4-hydroxylation de la debrisoquine a été démontrée (Clin. Pharmacol. Ther. 1991, *50*, 233-238). Le polymorphisme génétique du CYP 2D6 est responsable du métabolisme problématique de plus de 30 médicaments importants et affecte jusqu'à 10% de la population caucasienne (métaboliseurs lents). Il a été maintenant montré que cette isoforme contrôle la biotransformation de médicaments comme les anti-arythmiques, les β-bloquants, les anti-hypertenseurs, les antianginaux, les neuroleptiques et les antidépresseurs. A part quelques exceptions, ces médicaments sont utilisés en médecine psychiatrique et cardio-vasculaire pour le traitement à long terme.

Les conséquences pharmacocinétiques sont surtout d'ordre quantitatif : les sujets métaboliseurs lents ont un taux de produit inchangé plus élevé que les autres. Ces différences quantitatives ont un impact clinique considérable pour les molécules qui ont un index thérapeutique réduit.

La génétique influence donc fortement les différences d'efficacité et d'effets secondaires observés selon les individus. Ainsi, il est important de déterminer si le métabolisme d'un médicament peut-être modifié en cas de déficience génétique d'un enzyme.

Il a été maintenant trouvé, selon la présente invention, de nouveaux dérivés du benzène affins pour les récepteurs sigma notamment ceux du système nerveux périphérique, pourvus d'une activité immunosuppressive mais ayant un taux de métabolisation bas et/ou une implication nulle ou faible du CYP 2D6 dans le processus d'oxydation.

Les composés selon l'invention ont également une activité antitumorale, notamment ils inhibent la prolifération des cellules cancéreuses.

Par ailleurs, ces nouveaux composés se sont révélés avoir une activité dans le domaine cardiovasculaire, plus particulièrement sur le contrôle du rythme cardiaque.

Les composés selon l'invention ont également une activité sur l'apoptose.

Ainsi selon l'un de ses aspects, la présente invention concerne les composés de formule :

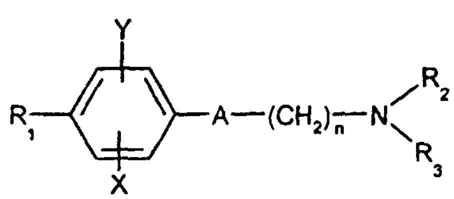

(I)

dans laquelle :

- A représente un groupe choisi parmi les suivants :
  -C≡C- , -CH=CH- ; $-CH_2-CH_2-$
- n est égal à 1 ou 2 ;
- X représente un atome d'hydrogène, de chlore ou de fluor ou un groupe méthyle ou méthoxy ;
- Y représente un atome d'hydrogène ou un atome de chlore ou de fluor ;
- $R_1$ représente un groupe cyclohexyle monosubstitué, disubstitué, trisubstitué ou tétrasubstitué par un groupe méthyle ; un groupe phényle monosubstitué ou disubstitué par un atome de fluor ou de chlore ou par un groupe méthoxy ; un groupe cycloheptyle, *tert*-butyle, dicyclopropylméthyle, bicyclo[3.2.1]octanyle, 4-tétrahydropyranyle, 4-tétrahydrothiopyranyle ou adamantyle 1 ou 2 ou adamantan-2-ol ; ou $R_1$ représente un groupe phényle étant entendu que dans ce cas X et Y sont différents de l'hydrogène ;
- $R_2$ représente un atome d'hydrogène ou un groupe $(C_1-C_4)$alkyle éventuellement substitué par un groupe trifluorométhyle;
- $R_3$ représente un $(C_5-C_7)$cycloalkyle ;

et les sels d'addition de ces composés avec des acides pharmaceutiquement acceptables, ainsi que leurs solvats et

hydrates.

Par alkyle, on entend un radical monovalent hydrocarboné, saturé, linéaire ou ramifié.

Par $(C_1-C_4)$alkyle, on entend un radical alkyle comprenant de 1 à 4 atomes de carbone.

Selon un autre de ses aspects, l'invention concerne les composés de formule (I) dans lesquels :

- A représente un groupe choisi parmi les suivants :
  $-C{\equiv}C-$ , $-CH{=}CH-$ ; $-CH_2-CH_2-$
- n est égal à 1 ou 2 ;
- X représente un atome d'hydrogène, de chlore ou de fluor ou un groupe méthyle ou méthoxy ;
- Y représente un atome d'hydrogène ou un atome de chlore ou de fluor ;
- $R_1$ représente un groupe cyclohexyle monosubstitué, disubstitué, trisubstitué ou tétrasubstitué par un groupe méthyle ; un groupe phényle monosubstitué ou disubstitué par un atome de fluor ou de chlore ou par un groupe méthoxy ; un groupe cycloheptyle, *tert*-butyle, dicyclopropylméthyle, bicyclo[3.2.1]octanyle, 4-tétrahydropyranyle, 4-tétrahydrothiopyranyle ou adamantyle 1 ou 2 ; ou $R_1$ représente un groupe phényle étant entendu que dans ce cas X et Y sont différents de l'hydrogène ;
- $R_2$ représente un $(C_1-C_4)$alkyle éventuellement substitué par un groupe trifluorométhyle;
- $R_3$ représente un $(C_5-C_7)$cycloalkyle ;

et les sels d'addition de ces composés avec des acides pharmaceutiquement acceptables, ainsi que leurs solvats et hydrates.

Selon un autre de ses aspects, l'invention concerne les composés de formule :

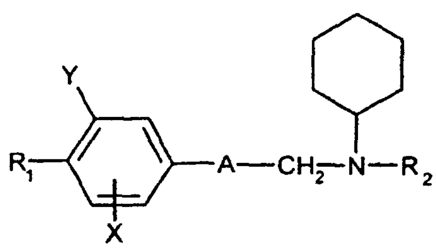 (I.1)

dans laquelle :

- A représente un groupe choisi parmi les suivants :
  $-C{\equiv}C-$ ; $-CH{=}CH-$ ; $-CH_2-CH_2-$
- X représente un atome d'hydrogène ou de chlore ;
- Y représente un atome d'hydrogène ou un atome de chlore;
- $R_1$ représente un cyclohexyle monosubstitué, disubstitué, trisubstitué ou tétrasubstitué par un groupe méthyle ; un groupe phényle substitué par un atome de chlore, un groupe méthoxy ou un ou deux atomes de fluor ; un groupe *tert*-butyle ou adamantyle 1 ou 2 ; ou $R_1$ représente un groupe phényle étant entendu que dans ce cas X et Y représentent tous les deux un atome de chlore ;
- $R_2$ représente un $(C_2-C_3)$alkyle ;

et les sels d'addition de ces composés avec des acides pharmaceutiquement acceptables, ainsi que leurs solvats et hydrates.

Selon un autre de ses aspects, l'invention concerne les composés de formule (I) et (I.1) dans lesquels A représente le groupe $-CH{=}CH-$ de configuration (Z) et les sels d'addition de ces composés avec des acides pharmaceutiquement acceptables, ainsi que leurs solvats et hydrates. Selon un autre de ses aspects, l'invention concerne les composés tels que définis ci-dessus dans lesquels X représente un atome de chlore et

Y représente un atome d'hydrogène ou de chlore et les sels d'addition de ces composés avec des acides pharmaceutiquement acceptables, ainsi que leurs solvats et hydrates.

Selon un autre de ses aspects, l'invention concerne les composés tels que définis ci-dessus dans lesquels $R_1$ représente le groupe 3,3,5,5-tétraméthylcyclohexyle ou 3,3-diméthylcyclohexyle ou 4,4-diméthylcyclohexyle, un groupe phényle monosubstitué ou disubstitué par un atome de fluor ou substitué en position 4 par un atome de chlore ; ou un groupe adamantyle 1 ou 2 ; et les sels d'addition de ces composés avec des acides pharmaceutiquement acceptables, ainsi que leurs solvats et hydrates.

Les composés suivants :

- [(Z)-3-(4-Adamantan-2-yl-3-chlorophényl)propèn-2-yl]cyclohexyléthylamine ;
- [(Z)-3-(4-Adamantan-2-ylphényl)propèn-2-yl]cyclohexyléthylamine ;
- {(Z)-3-[4-(4,4-Diméthylcyclohexyl)-2-chlorophényl]propèn-2-yl}cyclohexyléthylamine ;
- [(Z)-3-(4-Adamantan-1-yl-3-chlorophényl)propèn-2-yl]cyclohexyléthylamine ;
- [(Z)-3-(4-Adamantan-2-yl-3,5-dichlorophényl)propèn-2-yl]cyclohexyléthylamine ;
- [(Z)-3-(4-Adamantan-2-yl-3,5-dichlorophényl)propèn-2-yl]cyclohexyl(2-méthyléthyl) amine ;

ainsi que leurs sels avec des acides pharmaceutiquement acceptables, leurs solvats et hydrates constituent un autre aspect de l'invention.

En particulier, l'invention concerne le [(Z)-3-(4-Adamantan-2-yl-3,5-dichlorophényl) propèn-2-yl]cyclohexyléthylamine ainsi que ses sels avec des acides pharmaceutiquement acceptables, solvats et hydrates.

Les sels des composés selon l'invention sont préparés selon des techniques bien connues de l'homme de l'art.

Les sels des composés de formule (I) selon la présente invention comprennent ceux avec des acides minéraux ou organiques qui permettent une séparation ou une cristallisation convenable des composés de formule (I), ainsi que des sels pharmaceutiquement acceptables. En tant qu'acide approprié, on peut citer : l'acide picrique, l'acide oxalique ou un acide optiquement actif, par exemple un acide tartrique, un acide dibenzoyltartrique, un acide mandélique ou un acide camphosulfonique, et ceux qui forment des sels physiologiquement acceptables, tels que le chlorhydrate, le bromhydrate, le sulfate, l'hydrogénosulfate, le dihydrogénophosphate, le maléate, le fumarate, le 2-naphtalénesulfonate, le paratoluènesulfonate.

Parmi les sels des composés de formule (I), on préfère tout particulièrement les chlorhydrates.

Lorsqu'un composé selon l'invention présente un ou plusieurs carbones asymétriques, les isomères optiques de ce composé font partie intégrante de l'invention.

Lorsqu'un composé selon l'invention présente une stéréoisomérie par exemple de type axial-équatorial ou de type Z-E, l'invention comprend tous les stéréoisomères de ce composé.

La présente invention comprend les composés de formule (I) sous forme d'isomères purs mais également sous forme de mélange d'isomères en proportion quelconque. Les composés (I) sont isolés sous forme d'isomères purs par les techniques classiques de séparation : on pourra utiliser, par exemple des recristallisations fractionnées d'un sel du racémique avec un acide ou une base optiquement active dont le principe est bien connu ou les techniques classiques de chromatographies sur phase chirale ou non chirale, par exemple, on pourra utiliser la séparation sur gel de silice ou silice greffée $C_{18}$ en éluant avec des mélanges tels que solvants chloré/alcool. Les composés de formule (I) ci-dessus comprennent également ceux dans lesquels un ou plusieurs atomes d'hydrogène, de carbone ou d'halogène, notamment de chlore ou de fluor ont été remplacés par leur isotope radioactif par exemple le tritium ou le carbone-14. De tels composés marqués sont utiles dans des travaux de recherche, de métabolisme ou de pharmacocinétique, dans des essais biochimiques en tant que ligand de récepteurs.

Les groupes fonctionnels éventuellement présents dans la molécule des composés de formule (I) et dans les intermédiaires réactionnels peuvent être protégés, soit sous forme permanente soit sous forme temporaire, par des groupes protecteurs qui assurent une synthèse univoque des composés attendus. Les réactions de protection et déprotection sont effectuées selon des techniques bien connues de l'homme de l'art. Par groupe protecteur temporaire des amines, alcools, phénolthiols ou des acides carboxyliques on entend les groupes protecteurs tels que ceux décrits dans Protective Groups in Organic Synthesis, Greene T.W. et Wuts P.G.M., ed. John Wiley et Sons, 1991 et dans Protecting Groups, Kocienski P.J., 1994, Georg Thieme Verlag.

L'homme de l'art sera à même de choisir les groupes protecteurs appropriés. Les composés de formule (I) peuvent comporter des groupes précurseurs d'autres fonctions qui sont générées ultérieurement en une ou plusieurs étapes.

La présente invention a également pour objet un procédé pour la préparation des composés de formule (I) caractérisé en ce que :

1) dans le cas où A représente le groupement -C≡C- :

a) soit, si n = 1, on effectue une réaction de Mannich entre le dérivé phénylacétylénique de formule :

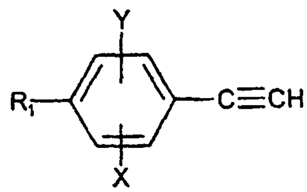

(II)

dans laquelle $R_1$, X et Y sont tels que définis pour (I), le formaldéhyde et l'amine (1) $HNR_2R_3$, $R_2$ et $R_3$ étant tels que définis pour (I) ;
b) soit on effectue un couplage de Suzuki entre le composé de formule :

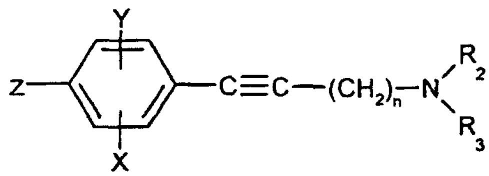

(Ia)

dans laquelle X, Y, n, $R_2$ et $R_3$ sont tels que définis pour (I) et Z représente un brome, un iode ou le groupe trifluorométhanesulfonate (OTf) et un dérivé boronique (2) de formule $R_1$-$B(OR)_2$ dans lequel R représente un atome d'hydrogène, un groupe alkyle ou aryle en présence d'une base et d'un catalyseur métallique ;
c) soit lorsque $R_1$ représente un groupe cyclohexyle monosubstitué, disubstitué, trisubstitué ou tétrasubstitué par un groupe méthyle ; un groupe cycloheptyle, 4-tétrahydropyranyle, 4-tétrahydrothiopyranyle ou adamantyle, on effectue un couplage entre le composé (Ia) dans lequel Z représente un atome d'iode ou de brome avec la cétone (3) correspondant à $R_1$ représentée par

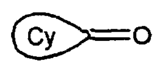

en présence d'une base pour obtenir intermédiairement le composé de formule :

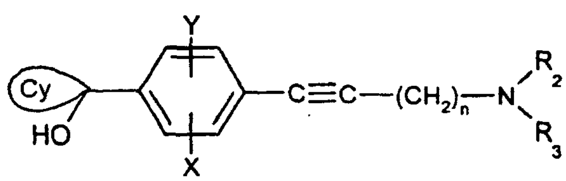

(I')

dans laquelle X, Y, n, $R_2$ et $R_3$ sont tels que définis pour (I) ; ledit composé (I') étant ensuite réduit dans des conditions sélectives ;
d) soit on effectue une réaction de couplage entre l'amine de formule :

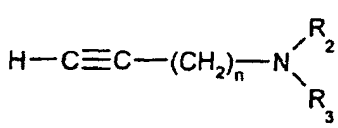

(4)

dans laquelle n, $R_2$ et $R_3$ sont tels que définis pour (I) et le composé de formule :

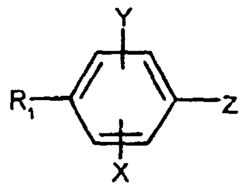

(III)

dans laquelle $R_1$, X et Y sont tels que définis pour (I) et Z représente un atome de brome, iode ou un groupe trifluorométhylsulfonate (triflate ou OTf) ;

2) dans le cas où A représente le groupe -CH=CH-, on effectue une hydrogénation par l'hydrogène naissant où en présence de cyclohexène du composé (I) dans lequel A représente le groupe acétylénique -C≡C- pour préparer le composé éthylénique (I) sous forme d'un mélange des isomères Z et E ou on effectue cette hydrogénation en présence d'un catalyseur métallique sur support pour préparer le composé éthylénique (I) sous forme Z, ou encore on fait réagir sur le composé (I) dans lequel A représente le groupe acétylénique -C≡C- un hydrure métallique pour préparer le composé éthylénique (I) sous forme E;

3) dans le cas où A représente le groupe $-CH_2-CH_2-$, on effectue une hydrogénation du composé (I) dans lequel A représente un groupe -CH=CH- ou -C≡C-.

A l'étape 1a du procédé selon l'invention, on procède à chaud, de préférence à une température comprise entre 80 et 90°C, dans un solvant polaire tel que le 1,2-diméthoxyéthane ou le 1,4-dioxane. Pour faciliter la réaction de condensation, on peut utiliser un catalyseur par exemple un sel métallique tel que le chlorure de cuivre II ou le chlorure de cuivre III.

A l'étape 1b du procédé, le couplage de Suzuki est de préférence effectué entre un composé (Ia) dans lequel Z représente OTf et le dérivé boronique (2) de formule $R_1-B(OH)_2$. La réaction est effectuée en présence d'une base, comme les hydroxydes, alcoxydes, phosphates ou carbonates alcalins ou alcalino terreux plus particulièrement le phosphate de potassium ou le carbonate de sodium. La réaction est effectuée en présence d'un catalyseur métallique, par exemple un catalyseur au cuivre, à l'étain ou de préférence au palladium tel que le tétrakis(triphénylphosphine) palladium éventuellement avec un halogénure du type chlorure de lithium jouant le rôle de cocatalyseur. On opère à chaud, à une température comprise entre 60 et 80°C dans un solvant inerte comme le toluène ou le 1,2-diméthoxyéthane ou de préférence en milieu biphasique toluène/solution aqueuse avec éventuellement une partie d'alcool comme l'éthanol.

Le couplage de Suzuki a été étudié dans de nombreuses publications comme par exemple Synth. Commun. 1981, *11* (7), 513-519 et J. Org. Chem. 1993, *58* (8), 2201-2208. Les acides boroniques (2) $R_1-B(OH)_2$ sont commerciaux ou synthétisés de façon classique à partir des dérivés halogénés correspondants de préférence bromés $R_1Br$ par action par exemple du triméthylborate en présence d'une base comme le *tert*-butyllithium.

A l'étape 1c, le couplage est de préférence effectué sur un composé (Ia) dans lequel Z représente un atome de brome, en présence d'une base comme le *n*-butyllithium dans un solvant inerte de préférence le éther diéthylique à basse température, -80 à -70°C étant la gamme de température préférée. La réduction de (I') en (I) est effectuée dans des conditions sélectives par exemple selon la méthode décrite dans Tetrahedron, 1995, *51*, 11043-11062 par action du chlorotriméthylsilane et de l'iodure de sodium dans un mélange acétonitrile/solvant chloré comme le dichlorométhane suivie d'un traitement à l'acide acétique en présence de zinc ou encore par action d'acide iodhydrique ou par hydrogénation ionique par action de tétraborohydrure de sodium dans l'acide triflique.

A l'étape 1d du procédé, le couplage est effectué en présence d'un catalyseur au palladium, d'une ou plusieurs amines tertiaires et éventuellement de chlorure de lithium. On préférera utiliser un composé (III) dans lequel Z représente un triflate et on opérera en présence d'un catalyseur au palladium du type tétrakis(triphényl phosphine)palladium ou dichlorodi(triphénylphosphine)palladium et éventuellement d'un cocatalyseur comme l'iodure de cuivre. Dans le cas où Z représente un triflate, on utilisera également le chlorure de lithium. Ce couplage est de préférence effectué en présence de triéthylamine et de pyridine à reflux du mélange réactionnel. Pour ce type de couplage, appelé couplage de Sonogashira, on pourra se référer à J. Org. Chem. 1993, *58*, 7368-7376 et 1998, *63*, 1109-1118 ; Syn. Lett. 1995, 1115-1116 et Synthesis, 1987, 981.

Pour préparer les composés (I) dans lesquels A représente le groupe -CH=CH- sous forme Z, on effectue en général l'hydrogénation en présence de cyclohexène et d'un catalyseur métallique sur support, tel que le palladium sur sulfate de baryum ou carbonate de calcium ou le nickel de Raney ou de préférence le catalyseur de Lindlar, dans un solvant inerte pour la réaction, comme l'éther de pétrole ou un solvant alcoolique. Pour préparer les composés (I) sous forme E, on préfère utiliser comme hydrure métallique l'hydrure de diisobutylammonium (DIBALH) dans un solvant inerte comme le toluène.

Pour préparer les composés (I) dans lesquels A représente le groupe $-CH_2-CH_2-$ l'hydrogénation est en général réalisée dans un alcool par exemple l'éthanol, en présence d'un catalyseur comme l'oxyde de platine ou de préférence le palladium sur charbon.

Pour les techniques de réduction des alcènes et alcynes utilisées ci-dessus, on pourra se référer à "Catalytic Hydrogenation. Techniques and Applications in Organic Chemistry", Robert L. Augustine, 1965, Marcel Dekker, Inc. New-York.

Le procédé général de préparation des composés (I) dans lesquels A représente le groupe acétylénique -C≡C- est décrit dans le SCHEMA 1 ci-après :

**SCHEMA 1**

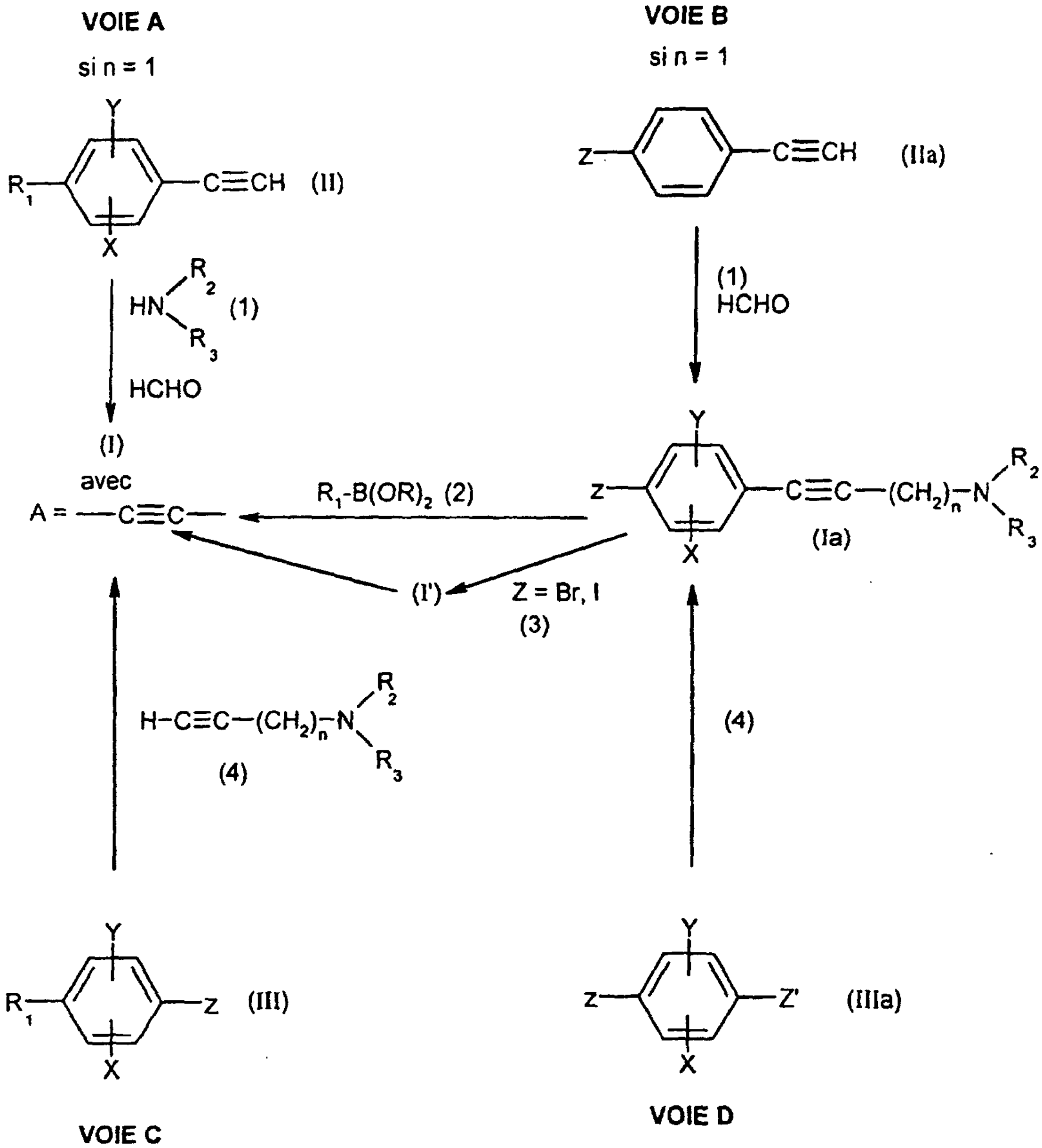


Dans le SCHÉMA 1, A = -C≡C-, et X, Y, n, $R_1$, $R_2$ et $R_3$ sont tels que définis pour (I), R représente un atome d'hydrogène, un groupe alkyle ou aryle, Z représente un atome de brome, d'iode ou un triflate et Z' représente un triflate lorsque Z représente un brome ou un iode sinon Z' représente un atome de brome ou d'iode. L'importance de la nature des substituants Z et Z' dans la réaction de couplage décrite VOIE D sera détaillée dans ce qui suit.

Le composé (II) est obtenu par traitement en milieu basique de la chloroacroléïne de formule :

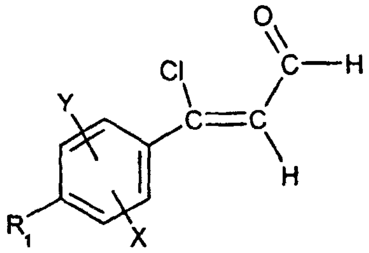

(IV)

dans laquelle X, Y et $R_1$ sont tels que définis pour (I), de préférence par action de l'hydroxyde de sodium dans un solvant comme le tétrahydrofurane ou de préférence le 1,4-dioxane à la température de reflux du solvant.

La chloroacroléïne (IV) est préparée à partir de l'acétophénone de formule :

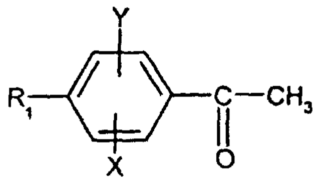

(V)

dans laquelle X,Y et $R_1$ sont tels que définis pour (I), par action d'un complexe de Vilsmeier. On utilise par exemple le chlorure de (chlorométhylène)diméthylammonium, complexe de Vilsmeier commercial, ou un complexe de Vilsmeier obtenu à partir d'une formamide disubstituée combinée avec le chlorure d'oxalyle, l'oxychlorure de phosphore ou le phosgène. On opère en général dans un solvant chloré ou un éther à une température comprise entre -20 et 40°C. Plus particulièrement, on utilise un complexe de Vilsmeier obtenu à partir du diméthylformamide et du chlorure d'oxalyle dans un solvant tel que le dichlorométhane ou le 1,2-diméthoxyéthane à des températures comprises entre -10 et 10°C.

Pour ce type de réaction, on pourra se référer par exemple à J. Chem. Soc. (C) 1970, 2484-2488 et Angew. Chem. Internat. Ed. 1963, 2, 98-99.

Les acétophénones (V) sont connues ou préparées selon des méthodes connues telles que celles décrites dans Gazz. Chim. Ital. 1949, 79, 453-457 et J. Am. Chem. Soc. 1947, 69, 1651-1652.

Le SCHEMA 2 illustre les méthodes utilisées pour la préparation des composés (V).

**SCHEMA 2**

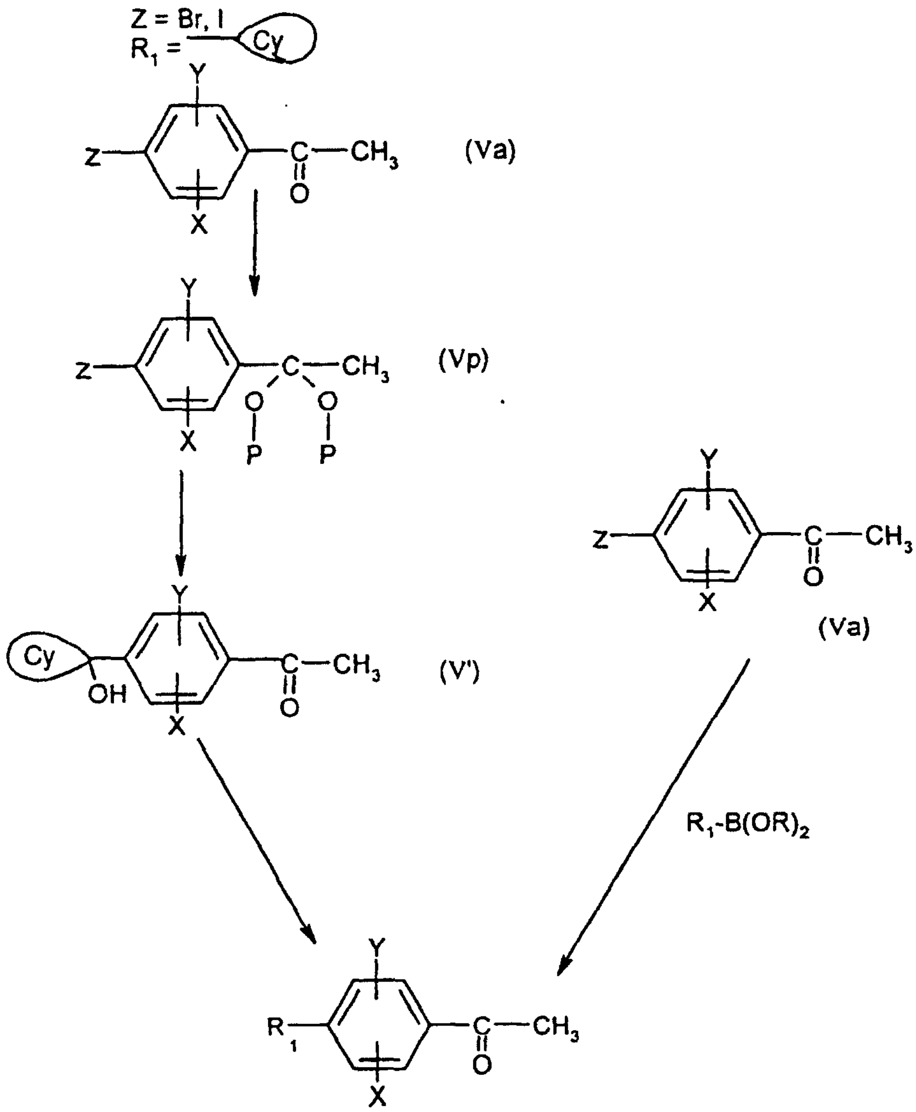


(Va)

(Vp)

(Va)

(V')

(V)

Dans le SCHEMA 2, X, Y et $R_1$ sont tels que définis pour (I), Cy est tel que défini précédemment pour (I'), Z représente un atome de brome, d'iode ou OTf, R représente un atome d'hydrogène, un groupe alkyle ou aryle et P représente un groupe protecteur de la fonction cétone comme un méthyle.

Les composés (V) peuvent être obtenus directement à partir des composés (Va) par action du composé boronique $R_1$-B(OR)$_2$ (2) comme décrit pour le passage de (Ia) à (I). On peut également protéger la fonction cétone du composé (Va) de manière classique par exemple par action d'orthoformate de trialkyle dans l'alcool correspondant en présence d'un acide comme l'acide *para*-toluènesulfonique. On obtient ainsi le composé (Vp)

que l'on fait réagir sur la cétone

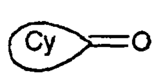

dans les conditions décrites pour le passage de (Ia) à (I'). On déprotège la fonction cétone par hydrolyse en milieu acide pour obtenir le composé (V'). Le dit composé (V') est alors réduit dans les conditions douces décrites pour le passage de (I') à (I).

Dans certains cas, par exemple, lorsque $R_1$ représente le groupe 4,4-diméthylcyclohexyle ou 4-tétrahydropyranyle, on pourra former intermédiairement le composé de formule :

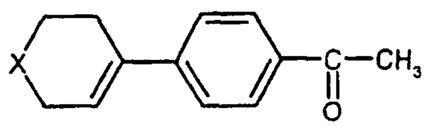

(VI)

dans laquelle X = O ou $-C(CH_3)_2$ qui conduira, après protection préalable de la fonction cétone, hydrogénation par exemple en présence de palladium sur charbon dans le méthanol, suivie de la déprotection de la fonction cétone, au composé (V) souhaité.

Les composés (V) dans lesquels X et/ou Y est différent de l'hydrogène peuvent être obtenus à partir des composés (V) dans lesquels X = Y = H par des méthodes connues de l'homme de l'art. Par exemple, dans le cas ou X et/ou Y représente un atome de chlore, on effectue la chloration du noyau aromatique par action de chlore gazeux en présence d'un acide de Lewis de préférence le trichlorure d'aluminium, dans un solvant chloré comme le dichlorométhane, de préférence à 0°C.

Les composés (Va) sont commerciaux ou peuvent être préparés selon des méthodes connues de l'homme du métier.

Par exemple, dans le cas ou Z représente un triflate, le composé (Va) peut être préparé comme présenté dans le SCHEMA 3 :

**SCHEMA 3**

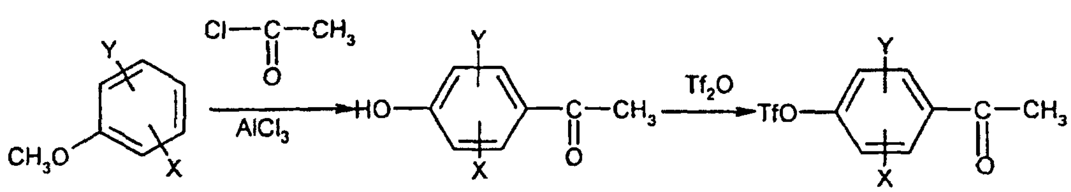


(VIII) (VII) (Va)

Dans le SCHEMA 3, X et Y sont tels que définis pour (I). Les composés (VIII) sont commerciaux ou préparés de manière classique.

Selon un autre de ses aspects, la présente invention a également pour objet les composés de formule (Ia) :

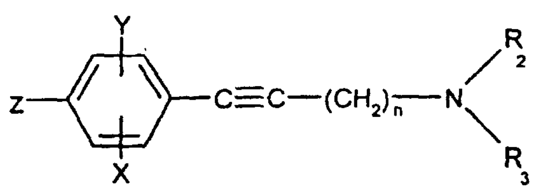

(Ia)

dans laquelle X, Y, n, $R_2$ et $R_3$ sont tels que définis pour (I) et Z représente un atome de brome, d'iode ou OTf. Ces composés sont nouveaux et constituent des intermédiaires clés dans la synthèse des composés (I).

La présente invention concerne également un procédé pour la préparation des dérivés (Ia) caractérisé en ce que :

- soit, lorsque n = 1, on effectue une réaction de Mannich entre le dérivé phénylacétylénique de formule :

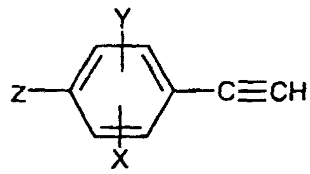

(IIa)

dans laquelle X et Y sont tels que définis pour (I) et Z représente un atome de brome , d'iode ou OTf, le formaldéhyde et l'amine (1) $HNR_2R_3$;

- soit on effectue une réaction de couplage entre l'amine de formule :

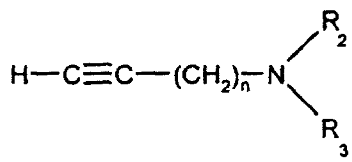

(4)

dans laquelle $R_2$, $R_3$ et n sont tels que définis pour (I) et le dérivé de formule :

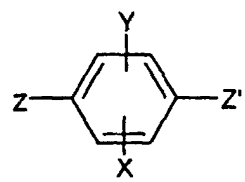

(IIIa)

dans laquelle X et Y sont tels que définis pour (I), Z représente un atome de brome, d'iode ou un triflate et Z' représente un atome de brome ou d'iode si Z représente un triflate sinon Z' représente un triflate, en présence d'un catalyseur au palladium, d'une ou plusieurs amines tertiaires et éventuellement de chlorure de lithium.

La réaction de Mannich est effectuée dans les mêmes conditions que celles décrites pour le passage de (II) à (I).

Pour le couplage entre les composés (IIIa) et (4) on utilise une réaction de Sonogashira décrite pour le couplage des composés (III) et (4). Lorsque Z représente un triflate et Z' un atome de brome ou d'iode, on opère en absence de chlorure de lithium. Par contre, lorsque Z représente un atome de brome ou d'iode et Z' un triflate, on opère en présence de chlorure de lithium. L'utilisation de chlorure de lithium permet d'orienter la réaction de couplage.

Les propargylamines (4) (cas ou n = 1), sont préparées de manière classique par exemple selon Tetrah. Lett. 1989, *30* (13), 1679-1682 à partir de l'amine (1) $HNR_2R_3$ et du 3-bromopropyne par action du carbonate de potassium dans l'acétonitrile à une température comprise entre 50 et 80°C.

Les composés (III) dans lesquels Z = OTf sont obtenus classiquement à partir des alcools correspondants de formule :

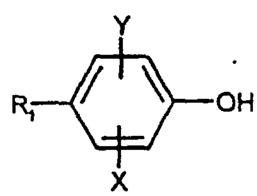

(IX)

dans laquelle X, Y et $R_1$ sont tels que définis pour (I), par action de l'anhydride trifluorométhanesulfonique (anhydride triflique) dans la pyridine.

Les alcools (IX) sont eux-mêmes obtenus à partir des composés de formule :

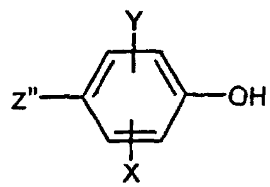

(IXa)

dans laquelle Z" représente un atome de brome ou d'iode selon les méthodes décrites précédemment pour le passage de (Ia) à (I) ou de (Va) à (V). Les composés (IXa) sont commerciaux ou préparés selon des techniques bien connues de l'homme de l'art.

Le composé (IIa) est préparé à partir de la chloroacroléine de formule :

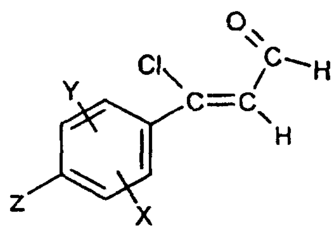

(IVa)

dans laquelle X, Y sont tels que définis pour (I) et Z représente un atome de brome, d'iode ou OTf, elle-même obtenue à partir de l'acétophénone de formule :

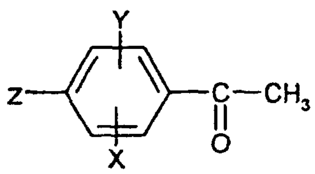

(Va)

dans laquelle X, Y et Z sont tels que définis précédemment pour (IVa) selon les méthodes décrites pour le passage de (IV) à (II) et de (V) à (IV).

Les composés selon l'invention ont fait l'objet d'études biochimiques et pharmacologiques. Les composés de formule (I) ainsi que leurs sels, hydrates et solvats pharmaceutiquement acceptables se lient spécifiquement aux récepteurs sigma, notamment à ceux du système nerveux périphérique, appelés également récepteurs sigma-2.

L'affinité pour les récepteurs sigma-1 a été étudiée *in vitro* sur des membranes de cerveau de cobaye en utilisant la $^{3}$H-(+)-3PPP comme ligand selon De Haven-Hudkins *et al.*, Life Science 1993, 53, 41-48. La (+)-pentazocine se fixe spécifiquement sur les récepteurs sigma-1. On prépare selon les méthodes usuelles un fragment de membrane de cerveau de cobaye. La préparation membranaire (0,3 mg de protéine/ml) est incubée pendant 150 minutes à 37°C en présence de 0,5 nM de [$^{3}$H]-(+)-pentazocine. La liaison non spécifique est déterminée en présence de 10 μM de (+)-pentazocine. Ensuite on filtre et rince 3 fois les membranes. On analyse la matière filtrée pour déterminer la fraction [$^{3}$H]-pentazocine spécifiquement liée. Dans ces conditions, les composés de l'invention, dont des exemples suivent, ont des $CI_{50}$ comprises entre 0,1 nM et 100 nM.

La capacité d'interaction des composés selon l'invention avec les récepteurs sigma-2 a été testée *in vitro* sur des membranes de rate de rat en utilisant comme ligand la [$^{3}$H]-DTG selon R. Paul *et al*, Journal of Neuroimmunology 1994, *52*, 183-192. La préparation membranaire (1 ml) est incubée avec 2 nM de [$^{3}$H]-DTG pendant 90 minutes à 20°C. La quantité de liaisons non spécifiques est estimée en présence de 10 μM de DTG ou d'halopéridol. Les membranes sont filtrées, lavées 2 fois et la matière filtrée est analysée pour déterminer la quantité de [$^{3}$H]-DTG spécifiquement liée. Les composés selon l'invention présentent une activité sigma-2 comprise entre 1 nM et 500 nM.

**1 - Les composés selon l'invention ont également été essayés dans des tests d'activité immunosuppressive exposés ci-après.**

D-galactosamine, SEB, LPS, proviennent de chez Sigma Chemical Co (St Louis, MO).

Le SEB contient moins de 0,00029% d'endotoxin (test "limulus amoebocyte lysate" Bioproduct, Walkersville,

MD). Ces molécules sont dissoutes dans une solution tampon de sel de phosphate ; les composés selon l'invention sont dissous dans une solution contenant 5% d'éthanol, 5% de Tween 80 et 90% d'eau.

Les souris utilisées sont des souris femelles Balb/C âgées de 6 à 8 semaines, provenant de l'élevage Charles River (France) et des souris femelles C57BL/6 et B6D2F1 âgées de 8 semaines provenant de l'élevage IFFA CREDO (Domaine des Oncins, BP 0109, 69592 L'Arbresle Cedex, France).

Détermination des cytokines : on injecte 5 souris avec les composés ou le solvant seul, par voie intrapéritonéale 30 minutes avant le LPS (10 µg/souris par voie intraveineuse) ou par voie orale 1 heure avant. On prélève des échantillons de sang par ponction rétro-orbitaire ou cardiaque, 1 heure 30 minutes après l'injection de LPS. On centrifuge les échantillons et prélève le sérum. Le sérum est stocké à -80°C avant analyse. On détermine les taux de TNF-α et IL-10 grâce au kit ELISA (Genzyme , Cambridge). Les essais sont menés d'après les instructions figurant dans la notice d'utilisation.

Choc toxinique: on administre à 10 animaux, les composés par voie intrapéritonéale. 30 minutes après, on leur administre le SEB (*Staphylococcus* enterotoxin B, Sigma St. Louis, MO) à raison de 10µg/souris par voie intraveineuse, la D-galactosamine (20 mg/souris, par voie intrapéritonéale).

La mort est constatée 48 heures après.

Maladie GVH (Graft-Versus-Host) ou réaction du greffon contre l'hôte : on injecte les composés à tester ou le solvant uniquement (pour contrôle) à des souris femelles B6D2F1 ($H2^b$ x $H2^d$) par voie intrapéritonéale. 4 heures après, on leur injecte 7,5 x $10^7$ cellules mononucléaires de rate de souris C57BL/6 ($H2^b$) pour amorcer la GVH. Tous les animaux sont sacrifiés 8 jours après la greffe et on mesure l'augmentation de poids de leur rate, causée par la GVH. L'indice suivant est calculé :

$$I = \frac{\text{poids de la rate (exp)}}{\text{poids de l'animal (exp)}} \Big| \frac{\text{poids de la rate (contr)}}{\text{poids de l'animal (contr)}}$$

Les résultats sont exprimés comme suit : PS = $\frac{\text{Iexp -1}}{\text{Icontr -1}}$ X 100
avec PS : pourcentage de splénomégalie.

Mesure de la prolifération des cellules T : Des suspensions cellulaires sont préparées à partir de rates de souris Balb/C. Les globules rouges sont tout d'abord lysés au cours d'un choc hypotonique court réalisé avec de l'eau distillée stérile. Les cellules restantes ( les globules blancs) sont lavés deux fois avec le milieu de culture (RPMI 1640 contenant 2% de sérum de veau foetal inactivé par la chaleur, 2mM de L-glutamine, 1mM de pyruvate de sodium, 100 UI/ml de pénicilline, 100 µg/ml de streptomycine et 15 mM de PIPES) préalablement ajusté à pH 6,6. La viabilité cellulaire, déterminée au moyen de la technique du bleu trypan excède toujours 95% par cette méthode de préparation.

Les splénocytes, à la concentration de 6 x $10^6$ cellules / ml, sont cultivés avec les produits à tester, dans des plaques 96 puits à fond plat (Falcon, Becton Dickinson, Lincoln Park, NJ) en présence de 2 µg/ml de SEB. Quatre puits sont effectués pour chaque concentration de produits à tester. L'incubation est réalisée à 37°C dans une étuve pour culture cellulaire (atmosphère : 95% d'air + 5% de $CO_2$) pendant 4 jours. 2 µCi de thymidine tritiée (Amersham, Les Ullis, France) sont alors ajoutés à chaque puits de la culture. Quatre heures après, les cellules sont récupérées sur un filtre en fibre de verre (Filtermat A, Wallac, Turku, Finland) au moyen d'un skatron (Pharmacia LKB, Piscataway, NJ). La radioactivité incorporée et fixée sur le filtre est mesurée dans un compteur à scintillation liquide adapté (Betaplate, Pharmacia LKB).

Les composés selon l'invention présentent donc, d'après les résultats observés lors de ces essais biochimiques et comportementaux une activité immunosuppressive.

**2 - Les composés selon l'invention ont également fait l'objet de tests montrant leur capacité à inhiber la prolifération de cellules tumorales et cancéreuses.**

Mesure de la prolifération des cellules MDA/MB231 (cancer du sein hormono-indépendant) : Les cellules MDA/MB231 sont entretenues *in vitro* par passages successifs dans le milieu DMEM (Gibco Laboratories, Grand Island, NY) contenant 10% de sérum de veau foetal inactivé par la chaleur, 1mM de pyruvate de sodium, 100 UI/ml de pénicilline et 100µg/ml de streptomycine.

Pour la mesure de prolifération, les cellules, à la concentration de 2 x $10^5$/ml, sont cultivées avec les produits à tester dans le milieu RPMI 1640 contenant 10µg/ml d'insuline bovine (Sigma) et 10µg/ml d'apotransferine (Sigma), dans des plaques 96 puits à fond plat (Falcon, Becton Dickinson, Lincoln Park, NJ). Trois puits sont effectués pour chaque concentration de produits à tester. L'incubation est réalisée à 37°C dans une étuve pour culture cellulaire (atmosphère : 95% d'air + 5% de $CO_2$) pendant 4 jours. 2µci de thymidine tritiée (Amersham, Les Ullis, France) sont alors ajoutés à chaque puits de la culture. Vingt quatre heures après, les cellules sont décollées au moyen de trypsine-EDTA (Gibco) et récupérées sur un filtre en fibre de verre (Filtermat A, Wallac, Turku, Finland) au moyen d'un skatron (Pharmacia LKB, Piscataway, NJ). La radioactivité incorporée et fixée sur le filtre est mesurée dans un compteur à scintillation liquide adapté (Betaplate, Pharmacia LKB).

**3 - Les composés selon l'invention ont également fait l'objet de tests démontrant leur intérêt dans le domaine cardiovasculaire.**

Les effets antiarythmiques des composés selon l'invention ont été testés sur les arythmies de reperfusion chez le rat anesthésié. L'expérimentation a été réalisée sur des rats normotendus mâles, Sprague Dawley, pesant de 250 à 300 g. Ces animaux proviennent de l'élevage IFFA CREDO. Les animaux sont maintenus dans des conditions de laboratoire standard se nourrissent avec de la nourriture standard : AO4 (UAR). L'eau est fournie *ad libitum*.

La technique d'occlusion et de reperfusion utilisée, dans cette étude, correspond aux méthodes décrites par Manning *et al*. (Circ. Res. 1984, 55, 545-548) et Kane *et al*. (Br. J. Pharmacol. 1984, *82*, 349-357), légèrement modifiées.

Les animaux ont été anesthésiés au pentobarbital sodique à 60 mg/kg par voie intrapéritonéale, trachéotomisés et ventilés avec de l'air ambiant (respirateur Harward). Un cathéter (PE10) a été placé dans la veine jugulaire pour l'injection intraveineuse des produits à tester. Des aiguilles hypodermiques ont été placées aux quatre pattes de l'animal pour enregistrer l'électrocardiogramme (ECG), le plus souvent DII (Gould ES1000 ou sur un polygraphe Astromed 7400). Après avoir réalisé une thoracotomie, un fil a été placé sur l'artère coronaire descendante antérieure gauche, près de son origine, pour préparer la ligature de l'artère. Les deux extrémités du fil sont passées à travers un tube en plastic, qui est placé sur la surface du coeur, juste au dessus de l'artère coronaire. L'occlusion de l'artère coronaire a été réalisée en exerçant une tension sur les extrémités du fil durant 5 minutes, et la reperfusion a été réalisée en relâchant la tension. La température de l'animal a été contrôlée et maintenue à 37°C grâce à une couverture homéothermique.

Pour l'étude de la voie intraveineuse, les produits ont été solubilisés dans un mélange de PEG 400/eau distillée à 75% et injectés 5 minutes avant de réaliser la ligature de la coronaire. Les produits ont été injectés dans un volume de 0,1 ml/100 g de rat. Le groupe contrôle a reçu ce solvant.

Pour l'étude de la voie orale, les produits ont été mis en suspension dans de la méthylcellulose à 0,6%, et administrés par gavage chez l'animal vigile, 120 minutes avant de réaliser la ligature de la coronaire. Les produits ont été administrés dans un volume de 1 ml/100 g de rat. Le groupe contrôle a reçu ce véhicule.

Les arythmies suivantes ont été analysées sur l'ECG pendant la période de reperfusion (étude durant 10 minutes), selon les Lambeth Conventions (Cardiovasc. Res., 1988, *22*, 447-455):

- extrasystoles ventriculaires (ESV),
- tachycardie ventriculaire (TV), sachant que la TV est la succession d'au moins quatre ESV,
- fibrillation ventriculaire (FV),
- et mortalité par fibrillation ventriculaire fatale ou par arrêt cardiaque.

Ces arythmies sont exprimées en pourcentage d'animaux présentant l'événement (fréquence).

Les animaux ont été répartis en groupe de 4 à 10 animaux. Chaque animal n'a reçu qu'une dose de produit.

Par voie intraveineuse comme par voie orale les produits protègent l'animal des arythmies de reperfusion en diminuant ou en supprimant la mortalité et la fréquence des FV. De plus certains produits diminuent et/ ou suppriment la fréquence des TV et des ESV, lorsqu'ils sont administrés par voie intraveineuse.

L'implication du CYP2D6 peut être mise en évidence par des études de métabolisme *in vitro* sur fractions microsomales hépatiques humaines. Le concept le plus utilisé est l'inhibition de l'enzyme par son inhibiteur spécifique : la quinidine utilisée à 20 fois son $K_i$ - $K_i$ étant la valeur absolue de la constante d'inhibition d'un principe actif vis-à-vis d'une enzyme.

Différents modèles permettent de mettre en évidence, dans une réaction métabolique spécifique, l'implication du CYP2D6.

- On peut utiliser des fractions microsomales hépatiques humaines qui contiennent l'ensemble des isoformes hépatiques humaines incubées en présence de co-facteur d'oxydoréduction (NADPH) et en absence ou en présence de quinidine à 20 fois son $K_i$ vis-à-vis du CYP2D6. La diminution de la métabolisation observée en présence de quinidine peut être associée à l'inhibition de l'isoforme CYP2D6, prouvant ainsi son éventuelle implication dans la ou les voies métaboliques étudiées.
- On peut également utiliser des fractions microsomales préparées à partir de cellules transfectées n'exprimant qu'une seule isoforme de cytochrome P-450 humaine (GENTEST Corp.).
- On peut également utiliser des hépatocytes humains en culture primaire qui sont capables d'effectuer des réactions

métaboliques de phase I et II. Les incubations sont alors réalisées en cinétique sur 24 heures en absence et en présence de quinidine, inhibiteur puissant et spécifique du CYP2D6. On pourra se référer à J. Pharm. Exp. Ther. 1996, 277, 321-332.

Les composés selon l'invention ont été particulièrement étudiés comme suit :

- le dit composé est incubé avec des fractions microsomales hépatiques humaines, du NADPH (co-facteur d'oxydo-réduction) ainsi qu'en présence ou en absence de quinidine. Le degré d'inhibition de la métabolisation observée en présence de quinidine reflète l'implication du CYP2D6 dans la métabolisation du dit composé. Cette approche est utilisable lorsque la métabolisation sur fractions microsomales hépatiques est d'une amplitude suffisante (c'est-à-dire supérieure ou égale à 10 % de la quantité de substrat de départ).
- Dans le cas où la métabolisation du dit composé sur microsomes hépatiques est trop faible pour pouvoir quantifier une inhibition avec précision, ou lorsque des vérifications supplémentaires sont nécessaires, des études complémentaires, plus approfondies, sur hépatocytes humains en culture primaire sont réalisées en cinétique sur 24 heures. Le degré d'implication du CYP2D6 dans la métabolisation hépatique globale est alors révélé par la diminution de la clairance intrinsèque du dit composé éventuellement observée en présence de quinidine.
- Les résultats obtenus montrent que les composés selon l'invention présentent un taux de métabolisation faible et/ou une faible implication du CYP2D6 dans le processus d'oxydation.

Aucun signe de toxicité n'est observé avec ces composés aux doses pharmacologiquement actives et leur toxicité est donc compatible avec leur utilisation comme médicaments.

Les composés de la présente invention sont particulièrement intéressants et pourront être utilisés avantageusement comme médicaments notamment pour traiter les maladies où il est souhaitable de réduire l'activité immunologique et également des maladies liées à des troubles inflammatoires. On peut citer à titre indicatif et non restrictif : les maladies à composantes auto-immune telles que par exemple, la polyarthrite rhumatoïde, le lupus érythémateux, les maladies dues à une démyélinisation comme la sclérose en plaque, la maladie de Crohn, les dermatites atopiques, le diabète ou bien les réactions de rejet de greffes, la réaction du greffon contre l'hôte, les situations de transplantation d'organes ou le psoriasis, ou encore les uvéites auto-immune, la maladie de Behçet et certaines spondylarthropathies, l'athérosclérose, l'asthme, les maladies fibrotiques, la fibrose idiopathique pulmonaire, la fibrose cystique, la glumérulonéphrite, la spondylite rhumatoïde, l'ostéoarthrite, la goutte, la résorbtion osseuse et cartilagineuse, l'ostéoporose, la maladie de Paget, l'uvéorétinite, les chocs septiques, la septicémie, les chocs endotoxiniques, le syndrome de détresse respiratoire de l'adulte, la silicose, l'asbestose, la sarcoïdose pulmonaire, la colite ulcérative, la sclérose latérale amyotrophique, la maladie d'Alzheimer, la maladie de Parkinson, le lupus érythémateux disséminé, les chocs hémodynamiques, les pathologies ischémiques (infarctus myocardique, ischémie myocardique, vasospame coronarien, angine de poitrine, insuffisance cardiaque, attaque cardiaque), les atteintes post ischémiques de reperfusion, la malaria, les infections mycobactériennes, la méningite, la lèpre, les infections virales (HIV, cytomegalovirus, virus de l'herpès), les infections opportunistes liées au Sida, la tuberculose, le psoriasis, la dermatose atopique et de contact, la cachexie, les dommages liés aux radiations.

Les composés selon l'invention peuvent aussi être utilisés en thérapie dans tous les processus pathologiques qui entraînent la prolifération des cellules tumorales. Cette prolifération cellulaire peut être soit hormono-sensible ou hormono-insensible. Plus précisément, des applications cliniques pour lesquelles on peut envisager l'utilisation de ces composés comprennent les maladies découlant d'une prolifération de cellules tumorales, en particulier des glioblastomes, des neuroblastomes, des lymphômes, des myélomes mélanomes, de la leucémie, des carcinomes du côlon, côlorectal, épithélial, hépatique, pulmonaire, mammaire, ovarien, pancréatique ou encore de la vessie ou de la prostate. Les composés selon l'invention pourront ainsi être avantageusement utilisés comme médicaments destinés à s'opposer à la prolifération des cellules tumorales, en particulier comme agents anti-tumoraux ou agents anti-cancéreux.

Ils pourront également être utilisé dans le domaine cardio-vasculaire, plus particulièrement pour traiter les troubles du rythme cardiaque.

Les composés selon l'invention peuvent également être très intéressants pour leur activité neuroprotectrice ainsi que pour leur activité sur l'apoptose.

L'utilisation des composés selon l'invention pour la préparation de médicaments destinés à traiter les maladies ci-dessus mentionnées fait partie intégrante de l'invention.

La présente invention a donc également pour objet des compositions pharmaceutiques contenant un composé selon l'invention ou un sel, solvat ou hydrate pharmaceutiquement acceptable de celui-ci, et des excipients convenables.

Lesdits excipients sont choisis selon la forme pharmaceutique et le mode d'administration souhaité.

Dans les compositions pharmaceutiques de la présente invention pour l'administration orale, sublinguale, sous-cutanée, intramusculaire, intra-veineuse, topique, intratrachéale, intranasale, transdermique, rectale ou intraocculaire, les principes actifs de formule (I) ci-dessus, ou leurs sels, solvats et hydrates éventuels, peuvent être administrés sous formes unitaires d'administration, en mélange avec des supports pharmaceutiques classiques, aux animaux et aux êtres humains pour la prophylaxie ou le traitement des troubles ou des maladies ci-dessus. Les formes unitaires d'administration appropriées comprennent les formes par voie orale telles que les comprimés, les gélules, les poudres, les granules et les solutions ou suspensions orales, les formes d'administration sublinguale, buccale, intratrachéale, intranasale, les formes d'administration sous-cutanée, intramusculaire ou intraveineuse et les formes d'administration rectale. Pour l'application topique, on peut utiliser les composés selon l'invention dans des crèmes, pommades, lotions ou collyres.

Afin d'obtenir l'effet prophylactique ou thérapeutique désiré, la dose de principe actif peut varier entre 0,2 et 15 mg par kg de poids du corps et par jour. Chaque dose unitaire peut contenir de 10 à 300 mg, de préférence de 25 à 75 mg, d'ingrédients actifs en combinaison avec un support pharmaceutique. Cette dose unitaire peut être administrée 1 à 5 fois par jour de façon à administrer un dosage journalier de 10 à 1500 mg, de préférence de 25 à 375 mg.

Lorsqu'on prépare une composition solide sous forme de comprimés, on mélange l'ingrédient actif principal avec un véhicule pharmaceutique, tel que la gélatine, l'amidon, le lactose, le stéarate de magnésium, le talc, la gomme arabique ou analogues. On peut enrober les comprimés de saccharose, d'un dérivé cellulosique, ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir ou pour l'administration sous forme de gouttes peut contenir l'ingrédient actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs, ou bien avec des matrices telles qu'un polymère ou une cyclodextrine (patch, formes à libération prolongée).

Les compositions de la présente invention peuvent contenir, à côté des produits de formule (I) ci-dessus ou de leurs sels, solvats et hydrates pharmaceutiquement acceptables, d'autres principes actifs qui peuvent être utiles dans le traitement des troubles ou maladies indiquées ci-dessus.

Ainsi, la présente invention a également pour objet des compositions pharmaceutiques contenant plusieurs principes actifs en association dont l'un est un composé selon l'invention.

Les PREPARATIONS et EXEMPLES suivants illustrent l'invention sans toutefois la limiter.

Les points de fusion ont été mesurés selon la technique Micro-köfler.

Les spectres de résonance magnétique nucléaire ont été effectués dans le diméthylsulfoxyde sauf mention contraire, à 200 MHz et les déplacements chimiques sont exprimés en p.p.m. Les abréviations utilisées ci-après sont les suivantes :

s = singulet ; m = multiplet ; d = doublet ; t = triplet ; q = quadruplet.

De manière conventionnelle, on numérotera par la suite le groupe phényle des composés (I) de la façon suivante :

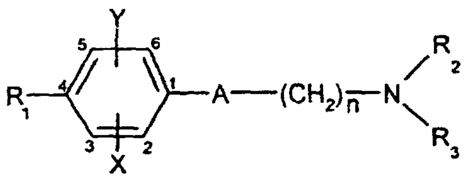

(I)

**PREPARATION 1**

**1-Bromo-4-(1,1-diméthoxyéthyl)benzène, *composé Vp***

(Vp) : X = Y = H ; Z = Br ; P = $CH_3$

On agite pendant 6 heures à température ambiante un mélange de 19,905 g de 1-(4-bromophényl)éthanone, 101,4 ml de méthanol, 0,22 g d'acide *para*-toluènesulfonique hydraté et 19,9 ml de triméthylorthoformate. On neutralise la solution avec une solution

d'hydroxyde de potassium à 1% dans le méthanol et concentre sous pression réduite. On reprend l'huile obtenue avec de l'éther de pétrole, élimine le précipité par filtration et évapore le filtrat sous pression réduite. On purifie le *composé IVp* par distillation ; rdt = 96% ; $T_{Eb}$ = 82°C (sous une pression de 0,03 mbar).

**PREPARATION 2**

**4,4-Diméthylcyclohexanone, composé 3.1**

a) 4,4-Diméthylcyclohex-2-ènone

A 81 ml de but-3-èn-2-one et 88 ml de 2-méthylpropionaldéhyde dans 450 ml de benzène, on additionne à température ambiante 1 ml d'acide sulfurique concentré, puis chauffe le mélange réactionnel à reflux pendant 13 heures pour éliminer l'eau par entraînement azéotropique. Après retour à température ambiante, le mélange réactionnel est lavé avec une solution aqueuse saturée en bicarbonate de sodium, puis à l'eau. La phase organique est séchée sur sulfate de magnésium et les solvants évaporés sous pression réduite. Après distillation, on isole 31,1 g du composé attendu ; $T_{Eb}$ = 78 °C (sous une pression de 22 mbar).

b) 31,1 g de 4,4-diméthylcyclohex-2-ènone dans 100 ml de pentane, sont hydrogénés en autoclave sous une pression de 5 bar en présence de 1,6 g de palladium sur charbon à 5%. Le mélange réactionnel est filtré et les solvants évaporés sous pression réduite.

**PREPARATION 3**

**4-Bromo-3,5-dichlorophénol, composé IXa.1**

a) *N*-(3,5-Dichlorophényl)acétamide

A 100 g de 3,5-dichlorophénylamine dans 3000 ml de chloroforme, on additionne goutte à goutte 200 ml de pyridine, puis 90 ml d'anhydride acétique. On agite le mélange réactionnel pendant 12 heures à température ambiante. Les solvants sont évaporés sous pression réduite et le résidu obtenu est recristallisé dans 1000 ml d'acétate d'éthyle ; F = 182°C.

b) *N*-(4-Bromo-3,5-dichlorophényl)acétamide

A 84,86 g de *N*-(3,5-dichlorophényl)acétamide et 34 g d'acétate de sodium dans 420 ml d'acide acétique, on additionne en 6 heures, 21,3 ml de brome dilué dans 82 ml d'acide acétique. Après 12 heures à température ambiante, on chauffe le mélange réactionnel pendant 5 heures à 50°C. On évapore les solvants sous pression réduite. Le résidu obtenu est recristallisé dans l'isopropanol ; F = 224°C.

c) 4-Bromo-3,5-dichlorophénylamine

202 g de *N*-(4-bromo-3,5-dichlorophényl)acétamide et 220 g d'hydroxyde de sodium (en solution aqueuse à 50%) dans 670 ml d'éthylèneglycol sont agités pendant 5 heures à 120°C, puis pendant 12 heures à température ambiante. On ajoute 3000 ml d'eau, filtre, sèche la phase organique sur sulfate de magnésium et évapore les solvants sous pression réduite. Le résidu obtenu est cristallisé dans le cyclohexane ; F = 132°C.

d)100 g de 4-bromo-3,5-dichlorophénylamine sont additionnés sous agitation à 5°C à un mélange de 125 ml d'eau et de 90 ml d'acide sulfurique concentré. On ajoute 230 g de glace pilée au mélange réactionnel, puis 29 g de nitrite de sodium dans 70 ml d'eau et laisse le mélange réactionnel 15 minutes sous agitation. On additionne rapidement le mélange réactionnel sur un mélange composé de 280 ml d'acide sulfurique concentré et de 200 ml d'eau porté à160°C et laisse le mélange réactionnel sous agitation à 160°C pendant 1 heure. On verse le mélange réactionnel sur un mélange eau/glace pilée et extrait au dichlorométhane. La phase organique est séchée sur sulfate de magnésium et les solvants sont évaporés sous pression réduite. Le résidu obtenu est purifié par chromatographie sur une colonne de gel de silice en éluant avec un mélange cyclohexane/dichlorométhane 4/6 (v/v).

$^1H$ RMN : 10,5(s,1H) ; 7,0(s,2H).

**PREPARATION 4**

**1-[4-(1-Hydroxy-3,3,5,5-tétraméthylcyclohexyl)phényl]éthanone, *composé V'.1***

(V'.1) : $R_1$ = 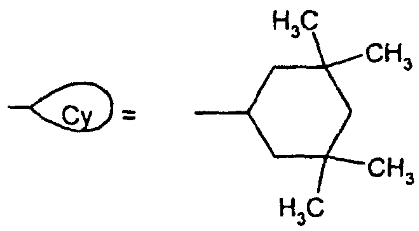 ; X = Y = H

A -78°C, à une solution de 10 g de 1-bromo-4-(1,1-diméthoxyéthyl)benzène (*composé Vp)* dans 100 ml de tétrahydrofurane, on ajoute goutte à goutte 27,5 ml d'une solution à 1,6 M dans l'hexane de *n*-butyllithium. On agite le mélange réactionnel pendant 2 heures à cette température. On ajoute en 20 minutes une solution de 6,92 ml de 3,3,5,5-tétraméthylcyclohexanone dans 20 ml de tétrahydrofurane et agite le mélange réactionnel à -78°C pendant 1 heure. Après retour à température ambiante on additionne 140 ml d'une solution aqueuse saturée en chlorure d'ammonium. On décante, extrait la phase aqueuse au éther diéthylique, on réunit les phases organiques, les sèche sur sulfate de magnésium et évapore les solvants sous pression réduite. On purifie l'huile obtenue par chromatographie sur une colonne de gel de silice en éluant avec un mélange cyclohexane / acétate d'éthyle, 95 / 5 (v/v) ; rdt = 88% ; F = 135°C.

De la même manière, on prépare les composés suivants :

**1-[4-(Hydroxy-3,3-diméthylcyclohexyl)phényl]éthanone, *composé V'.2***

(V'.2) : $R_1$ = 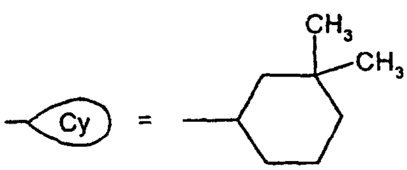 ; X = Y = H

F = 99°C.

**1-[4-(Hydroxyadamantan-2-yl)phényl]éthanone, *composé V'.3***

(V'.3) : $R_1$ = 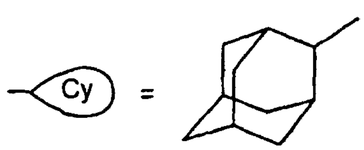 ; X = Y = H

$^1H$ RMN : 7,9 (d,2H) ; 7,6 (d,2H) ; 4,8 (s,1H) ; 2,6-1,4 (m, 18H).

**1-[4-(Hydroxy-4,4-diméthylcyclohexyl)phényl]éthanone, *composé V'.4***

(V'.4) : $R_1$ = 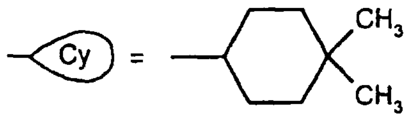 ; X = Y = H

F = 88°C.

**PREPARATION 5**

**1-[4-(3,3,5,5-Tétraméthylcyclohexyl)phényl]éthanone, *composé V.1***

(V.1) : $R_1$ = 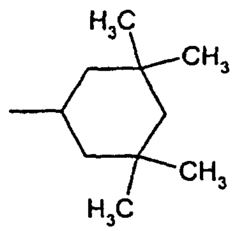 ; X = Y = H

A une solution de 40,45 g de 1-[4-(hydroxy-3,3,5,5-tétraméthylcyclohexyl)phényl] éthanone (*composé V'.1)* et de 56,21 g d'iodure de sodium dans 230 ml d'acétonitrile anhydre, on additionne 38,1 ml de chlorotriméthylsilane en 45 minutes. Pendant l'addition, on maintient la température entre 35 et 40°C. Après 2 heures d'agitation, on additionne 40 ml d'acétonitrile et 39,4 ml d'acide acétique. Ensuite, on ajoute par portions 29,4 g de zinc en poudre fine, sous agitation et à température ambiante. On chauffe à reflux sous forte agitation pendant 4 heures. Après retour à température ambiante, on filtre sur célite puis lave avec une solution aqueuse saturée en bicarbonate de sodium. On concentre la phase organique sous pression réduite et purifie l'huile obtenue par chromatographie sur une colonne de gel de silice en éluant avec un mélange cyclohexane / acétate d'éthyle , 95/5 (v/v) ; rdt = 68% ; F = 54°C.
De la même manière, on prépare les composés suivants :

**1-[4-(3,3-Diméthylcyclohexyl)phényl]éthanone, *composé V.2***

(V.2) : $R_1$ = 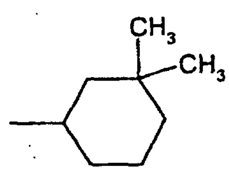 ; X = Y = H

$^1$H RMN : 7,8 (d,2H) ; 7,2 (d,2H) ; 2,7 (m,1H) ; 2,5 (s,3H) ; 1,8-1,1 (m,8H) ; 1,0 (s,3H) ; 0,9 (s,3H).

**1-(4-Adamantan-2-ylphényl)éthanone, composé V.3**

(V.3) : $R_1$ = 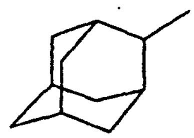 ; X = Y = H

F = 75°C.

**PREPARATION 6**

**1-[4-(4,4-Diméthylcyclohex-1-ényl)phényl]éthanone, composé VI.1**

a) 1-[4-(1,1-Diméthoxyéthyl)phényl]-4,4-diméthylcyclohexanol

A 117 g de 1-bromo-4-(1,1-diméthoxyéthyl)benzène dans 1100 ml de tétrahydrofurane, on additionne à -78°C 328 ml d'une solution 1,6M de butyllithium dans le cyclohexane et agite le mélange réactionnel à -78°C pendant 2 heures. A cette même température, on additionne 66 g de 4,4-diméthylcyclohexane en solution dans 210 ml de tétrahydrofurane et agite le mélange réactionnel pendant 1 heure à -78°C. Le mélange réactionnel est hydrolysé par ajout de glace pilée. La phase organique est décantée, séchée sur sulfate de sodium et les solvants sont évaporés sous pression réduite. Le composé obtenu est recristallisé dans 500 ml de n-hexane ; F = 88°C.

b) Sous atmosphère inerte, à 600 ml d'acétonitrile, on additionne 99,32 g de 1-[4-(1,1-diméthoxyéthyl)phényl]-4,4-diméthylcyclohexanol dans 300 ml de dichlorométhane, 151 g d'iodure de sodium et chauffe le mélange réactionnel à 30°C. On additionne 102 ml de chlorure de chlorotriméthylsilane, puis à 65°C on ajoute par portions un mélange de 300 ml d'acétonitrile et de 47 ml d'acide acétique et agite le mélange réactionnel pendant 12 heures à température ambiante. Le mélange réactionnel est filtré et extrait au dichlorométhane. Le résidu obtenu est purifié par chromatographie sur une colonne de gel de silice en éluant avec un mélange cyclohexane/acétate d'éthyle 99/1 (v/v).

**PREPARATION 7**

**1-[4-(4,4-Diméthylcyclohexyl)phényl]éthanone, composé V.4**

a) 1-(1,1-Diméthoxyéthyl)-4-(4,4-diméthylcyclohex-1-ényl)benzène

On agite pendant 12 heures à température ambiante 36,13 g de 1-[4-(4,4-diméthylcyclohex-1-ényl)phényl] éthanone (*composé VI.1*) dans 250 ml de méthanol, en présence de 0,5 g d'acide para-toluènesulfonique (APTS) et de 13 ml de triméthyl-*ortho*-formiate. Les solvants sont partiellement évaporés sous pression réduite. Une solution d'hydroxyde de potassium dans le méthanol à 50% est additionnée puis les solvants sont évaporés sous pression réduite. Le résidu obtenu est repris à l'éther diisopropylique, puis le solvant est évaporé sous pression réduite.

b) le composé obtenu en a) dans 250 ml de méthanol, est hydrogéné en présence de 3 g de palladium sur charbon à 5%. Le mélange réactionnel est filtré, les solvants sont évaporés sous pression réduite et le résidu obtenu est repris au dichlorométhane. On laisse le mélange réactionnel sous agitation pendant 12 heures en présence de silice, filtre, évapore les solvants sous pression réduite et purifie le résidu obtenu par chromatographie sur une colonne de gel de silice en éluant avec un mélange cyclohexane/acétate d'éthyle 99/1 (v/v) ; F = 60°C.

**PREPARATION 8**

**1-[3-Chloro-4-(3,3,5,5-tétraméthylcyclohexyl)phényl]éthanone,** ***composé V.5***

(V.5) : $R_1$ = 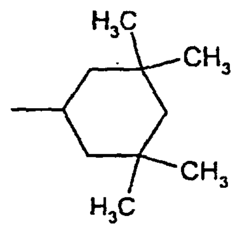 ; X = 3-Cl ; Y = H

Sous atmosphère inerte, à 350 ml de dichlorométhane on additionne à 0°C 40,25 g de chlorure d'aluminium puis 5 g de 1-[4-(3,3,5,5-tétraméthylcyclohexyl)phényl]éthanone (*composé V.1)* en solution dans du dichlorométhane. Après 2 heures d'agitation à 0°C on fait barboter dans la réaction 17,1 ml de chlore gazeux (d = 1,565, mesuré à l'état liquide à -78°C). Après retour à température ambiante, on additionne au mélange réactionnel un mélange eau / glace. On extrait au dichlorométhane, décante, sèche la phase organique sur sulfate de magnésium et concentre sous pression réduite. On purifie sur une colonne de gel de silice en éluant avec un mélange cyclohexane/dichlorométhane, 7/3 (v/v) ; rdt = 74% ; F = 64°C.

On isole également les composés dichlorés suivants par chromatographie :

**1-[3,5-Dichloro-4-(3,3,5,5-tétraméthylcyclohexyl)phényl]éthanone,** ***composé V.6***

(V.6) : $R_1$ = 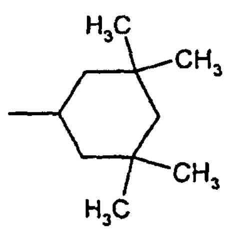 ; X= 3-Cl ; Y = 5-Cl

$^1$H RMN : 7,9 (s,1H) ; 7,8 (s,1H) ; 3,9 (m,1H) ; 2,5 (s,3H) ; 2,1 (m,2H) ; 1,2 (m,4H) ; 1,0 (s,6H) ; 0,9 (s,6H).

**1-[3,6-Dichloro-4-(3,3,5,5-tétraméthylcyclohexyl)phényl]éthanone,** ***composé V.7***

(V.7) : $R_1$ = 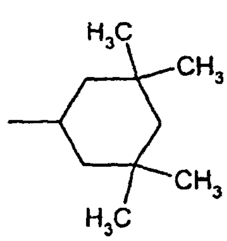 ; X= 3-Cl ; Y = 6-Cl

$^1$H RMN : 7,6 (s,1H) ; 7,2 (s,1H) ; 3,3 (m,1H) ; 2,6 (s,3H) ; 1,5 (m,2H) ; 1,2 (m,4H) ; 1,1 (s,6H) ; 0,9 (s,6H).

Selon le mode opératoire décrit pour le *composé V.5*, on isole les composés suivants :

**1-[3-Chloro-4-(3,3-diméthylcyclohexyl)phényl]éthanone, composé *V.8***

(V.8) : $R_1$ = 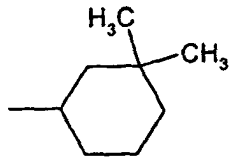 ; X = 3-Cl ; Y = H

$^1$H RMN : 7,9 (1H,s) ; 7,8 (d,1H) ; 7,4 (d,1H) ; 3,1 (m,1H) ; 2,5 (s,3H) ; 1,8-1,1 (m,8H) ; 0,9 (s,3H) ; 0,8 (s,3H).

**1-(3-Chloro-4-*tert*-butylphényl)éthanone, *composé V.9***

(V.9) : $R_1$ = 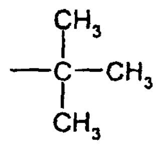 ; X = 3-Cl ; Y = H

$^1$H RMN : 7,8 (s,1H) ; 7,7 (d,1H) ; 7,5 (d,1H) ; 2,5 (s,3H) ; 1,4 (s,9H).

**1-(3,5-Chloro-4-cyclohexylphényl)éthanone, *composé V.10***

(V.10) : $R_1$ = 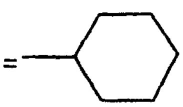 ; X = 3-Cl ; Y = 5-Cl

**1-[3-Chloro-(4,4-diméthylcyclohexyl)phényl]éthanone, *composé V.11***

(V.11) : $R_1$ = 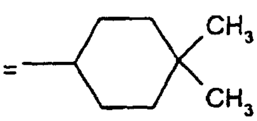 ; X = 3-Cl ; Y = H

$^1$H RMN : 7,9(s,1H) ; 7,8(d,1H) ; 7,5(d,1H) ; 2,8(m,1H) ; 2,5(s,3H) ; 1,8-1,1(m,8H) ; 0,95(s,3H) ; 0,9(s,3H)

**PREPARATION 9**

**1-[(3-Chloro-4-hydroxy)phényl]éthanone, *composé VII.1***

(VII.1) : X = 3-Cl ; Y = H

Sous atmosphère inerte, à 63,5 ml de 2-chloro-1-méthoxybenzène dans 500 ml de 1,2-dichloroéthane, on additionne 167 g de trichlorure d'aluminium puis on ajoute goutte à goutte 167 g de chlorure d'acétyle en solution dans 200 ml de 1,2-dichloroéthane. On chauffe le mélange réactionnel à 45°C pendant 48 heures. On verse le mélange réactionnel sur un mélange eau/glace, extrait au dichlorométhane, évapore les solvants sous pression réduite et purifie

le résidu obtenu par chromatographie sur une colonne de gel de silice en éluant avec un mélange cyclohexane/acétate d'éthyle, 90/10 (v/v). On recristallise le *composé VII.1* dans le cyclohexane ; F = 107°C.

**PREPARATION 10**

**Cyclohexyléthylprop-2-ynylamine, *composé (4.1)***

A 30,3 ml de cyclohexyléthylamine et 29,7 g de carbonate de potassium dans 300 ml d'acétonitrile, on additionne goutte à goutte 20 ml de 3-bromopropyne à 80%. On chauffe le mélange réactionnel à 50°C pendant 12 heures et 6 heures à 80°C. On filtre, évapore les solvants sous pression réduite. Le *composé V.1* est purifié par distillation.
$^{1}$ H RMN : 3,3 (s,2H) ; 3,0 (s,1H) ; 2,5 (q,2H) ; 2,4 (m,1H) ; 1,8-1,1 (m,10H) ; 1,0 (t,3H). De la même manière, on prépare les composés suivants :

**Cyclohexylméthylprop-2-ynylamine,** *composé 4.2*
**Cyclohexylisopropylprop-2-ynylamine,** *composé 4.3*

**PREPARATION 11**

**Cyclohexyléthylbut-3-ynylamine, *composé (4.4)***

a) But-3-yne(4-méthylphényl)sulfonate
A 36 ml de pyridine, on additionne à 80°C, 74,8 g de chlorure de tosyle. On refroidit le mélange réactionnel à 15°C, puis additionne 25 g de but-3-yn-1-ol. On agite le mélange réactionnel à température ambiante pendant 12 heures puis additionne à 15°C 70 ml d'eau, extrait à l'éther diéthylique puis lave la phase organique avec une solution aqueuses d'acide sulfurique dilué puis avec une solution aqueuse saturée en hydrogénocarbonate de sodium. La phase organique est séchée sur sulfate de sodium et les solvants évaporés sous pression réduite.
$^{1}$H RMN : 7,8(d,2H) ; 7,4(d,2H) ; 4,0(t,2H) ; 3,8(s,1H) ; 2,5(t,2H) ; 2,4(s,3H)

b) On chauffe à reflux pendant 12 heures 57,9 g du composé obtenu en a), 21,7 g d'hydrogénocarbonate de sodium, 35,7 ml de cyclohexyléthylamine dans 100 ml de diméthylformamide. Le mélange réactionnel est versé sur de l'eau et extrait à l'éther diéthylique. On sèche la phase organique sur sulfate de magnésium et évapore les solvants sous pression réduite. Après distillation, on isole l'amine attendue ; $T_{eb}$ = 92 - 94°C (sous une pression de 13mbar).

**PREPARATION 12**

**4-Acétyl-2-chlorophényltrifluorométhanesulfonate, *composé Va.1***

(Va.1) : X = 3-Cl ; Y = H ; Z = OTf

A 0°C, à 26,7 g de 1-[(3-chloro-4-hydroxy)phényl]éthanone (*composé VII.1)* dans 700 ml de pyridine, on additionne goutte à goutte 26,2 ml d'anhydride triflique. On agite le mélange réactionnel à 0°C pendant 36 heures, évapore les solvants sous pression réduite et reprend le résidu avec une solution 0,1 N d'acide chlorhydrique dans le dichlorométhane. On décante, sèche les phases organiques sur sulfate de magnésium et évapore les solvants sous pression réduite. Le résidu obtenu est purifié par chromatographie sur une colonne de gel de silice en éluant avec un mélange cyclohexane/acétate d'éthyle , 95/5 (v/v).
$^{1}$H RMN : 8,2 (s,1H) ; 8,0 (d,1H) ; 7,8 (d,1H).
De la même manière on prépare les composés suivants :
**4-Acétyl-2,6-dichlorophényltrifluorométhanesulfonate, *composé Va.2***
(Va.2) : X = 3-Cl ; Y = 6-Cl ; Z = OTf
$^{1}$H RMN : 8,2 (s,2H) ; 2,6 (s,3H).
**4-Bromo-2-chlorophényltrifluorométhanesulfonate , *composé IIIa.1*** à partir du 4-**bromo-2-chlorophénol.**
(IIIa.1) : X = 3-Cl ; Y = H
$^{1}$H RMN : 8,1 (s,1H) ; 7,7 (d,1H) ; 7,6 (d,1H).

**PREPARATION 13**

**2-Chloro-4-[3-(cyclohexyléthylamino)prop-1-ynyl]phényltrifluorométhane sulfonate, *composé Ia.1***

(Ia.1) : Z = OTf ; X = 3-Cl ; Y = H; 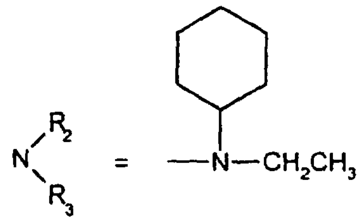

Sous atmosphère inerte, à 4 g de 4-bromo-3-chlorophényltrifluorométhanesulfonate (*composé IIIa.1)*, 0,06 g d'iodure de cuivre, 10 ml de pyridine et 20 ml de triéthylamine, on additionne 2,14 g de cyclohexyléthylprop-2-ynylamine (*composé VII.1)* puis 0,413 g du catalyseur dichlorobis(triphénylphosphine)palladium VI . On chauffe le mélange réactionnel à reflux pendant 2 heures puis à température ambiante pendant 12 heures. On filtre, évapore les solvants sous pression réduite. Le résidu est purifié par chromatographie sur une colonne de gel de silice en éluant avec un mélange dichlorométhane/éthanol variant de 100/0 à 99/1 (v/v). On reprend le composé obtenu avec du dichlorométhane, filtre, évapore les solvants sous pression réduite ; rdt = 76% $^{1}$H RMN : 7,8 (s,1H) ; 7,6 (d,1H) ; 7,5 (d,1H) ; 3,6 (s,2H) ; 2,6 (q,2H) ; 2,4 (m,1H) ; 1,9-1,1 (m,10H) ; 0,9 (t,3H).

**PREPARATION 14**

**1-[3-Chloro-4-(4-fluorophényl)phényl]éthanone, *composé V.12***

(V.12) : $R_1$ = 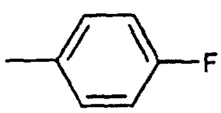 ; X = 3-Cl ; Y= H

Sous atmosphère inerte, on agite à 60°C pendant 8 heures 19,7 g de 4-acétyl-2-chlorophényltrifluorométhanesulfonate (*composé X.1*), 10 g d'acide 4-fluorobenzèneboronique, 2 g de tétrakis(triphénylphosphine)palladium, 17,9 g de carbonate de sodium dans 84,5 ml d'eau, 591 ml de toluène, 200 ml d'éthanol et 5,51 g de chlorure de lithium. On agite ensuite le mélange réactionnel pendant 12 heures à température ambiante. On filtre, évapore les solvants du filtrat sous pression réduite. Le résidu obtenu est purifié par chromatographie sur une colonne de gel de silice en éluant avec un mélange cyclohexane/acétate d'éthyle, 97/3 (v/v) ; rdt = 94%.
$^{1}$H RMN : 8,0 (s,1H) ; 7,9 (d,1H) ; 7,5 (m,3H) ; 7,3 (m,2H) ; 2,6 (s,3H).

De la même manière, on prépare les *composés V.13* à *V.17* présentés dans le TABLEAU 1 suivant :

**TABLEAU 1**

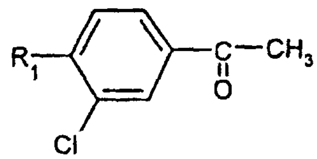

(V)

| COMPOSE | $R_1$ | $^1H$ RMN |
|---|---|---|
| *V.13* | 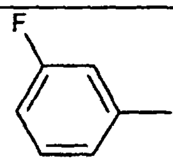 | 8,1 (s,1H) ; 7,9 (d,1H) ; 7,5 (m,2H) ; 7,2 (m,3H) ; 2,6 (s,3H) |
| *V.14* | 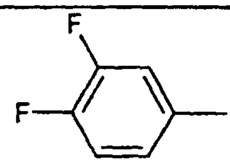 | 8,0 (s,1H) ; 7,9 (d,1H) ; 7,6 (m,3H) ; 7,3 (m,1H) ; 2,6 (s,3H) |
| *V.15* | 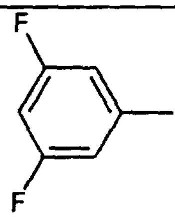 | 8,0 (s,1H) ; 7,9 (d,1H) ; 7,6 (d,1H) ; 7,4-7,1 (m,3H) 2,6 (s,3H) |
| *V.16* | 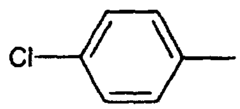 | 8,0 (s,1H) ; 7,9 (d,1H) ; 7,5 (m,5H) ; 2,6 (s,3H) |

**TABLEAU 1 (suite 1)**

| COMPOSE | $R_1$ | $^1H$ RMN |
|---|---|---|
| *V.17* | 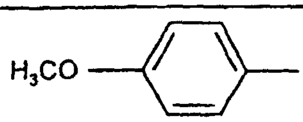 | 8,0 (s,1H) ; 7,9 (d,1H) ; 7,5 (d,1H) ; 7,4 (m,2H) ; 7,0 (m,2H) ; 3,8 (s,3H) ; 2,6 (s,3H) |

**1-(2,6-Dichlorobiphényl-4-yl)éthanone, composé *V.18***

(V.18) : $R_1$ = 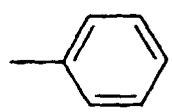 ; X = 3-Cl ; Y = 5-Cl

$^1H$ RMN : 8,0 (s,2H) ; 7,4 (m,3H) ; 7,2 (m,2H) ; 2,6 (s,3H).

**1-(2,6-Dichloro-4'-fluorobiphényl-4-yl)éthanone, *composé V.19***

(V.19) : $R_1$ = 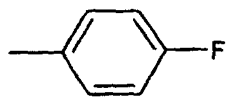 ; X = 3-Cl ; Y = 5-Cl

$^1H$ RMN : 8,0 (s,2H) ; 7,3 (m,4H) ; 2,6 (s,3H).

**PREPARATION 15**

**3-Chloro-3-[3-chloro-4-(3,3,5,5-tétraméthylcyclohexyl)phényl]propénal, *composé IV.1***

(IV.1) $R_1$ = 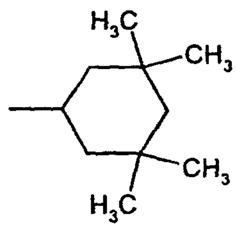 ; X = 3-Cl ; Y = H

A une température comprise entre -5 et 2°C, à une solution de 3,72 ml de diméthylformamide et de 20 ml de dichlorométhane anhydre, on ajoute goutte à goutte 3,51 ml de chlorure d'oxalyle, puis agite le mélange réactionnel pendant 30 minutes à température ambiante. On additionne alors rapidement 3,92 g de 1-[3-chloro-4-(3,3,5,5 -tétraméthylcyclohexyl)phényl]éthanone (*composé V.6)* en solution dans 10 ml de dichlorométhane, puis on agite le mélange réactionnel à température ambiante pendant 12 heures. On verse le mélange réactionnel sur un mélange eau/glace, puis on ajoute 20 ml d'une solution aqueuse d'éthanoate de sodium à 2,84 M. On lave avec 50 ml d'une solution d'hydrogénocarbonate de sodium et 50 ml d'eau, décante, sèche la phase organique sur sulfate de magnésium et évapore les solvants sous pression réduite. On purifie l'huile obtenue par chromatographie sur une colonne de gel de silice en éluant avec un mélange cyclohexane/acétate d'éthyle, 97/3 (v/v).
$^1H$ RMN : 10,2 (d,1H) ; 7,7 (s,1H) ; 7,5 (d,1H) ; 7,3 (d,1H) ; 6,6 (d,1H) ; 3,4 (m,1H) ; 1,5 (m,2H) ; 1,3 (m,4H) ; 1,1 (s, 6H) ; 0,9 (s,6H).

De la même manière, on prépare les composés *IV.2 à IV.17* présentés dans les TABLEAUX 2 et 3 ci-après :

## TABLEAU 2

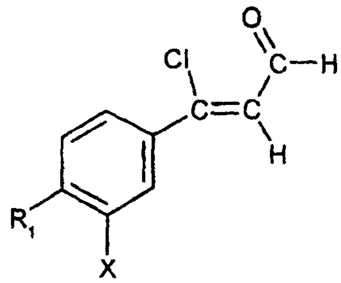

(IV) avec Y = H

| COMPOSE | $R_1$ | X | F ; °C ou $^1H$ RMN |
|---|---|---|---|
| *IV.2* | 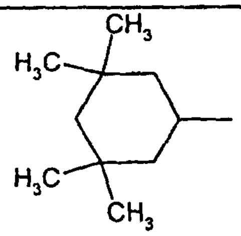 | H | 10,1 (d,1H) ; 7,8 (m,2H); 7,4 (m,2H) ; 6,9 (m,1H); 2,9 (m,1H) ; 1,4-0,8 (18H) |
| *IV.3* | 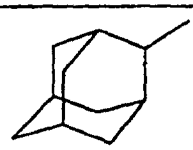 | H | 146 |
| *IV.4* | 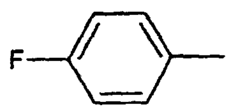 | H | |
| *IV.5* | 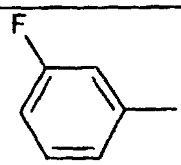 | H | |
| *IV.6* | 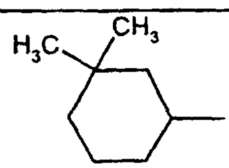 | Cl | 10,0(d,1H) ; 7,8(s,1H);7,7(d,1H) ; 7,4(d,1H) ; 7,0(d,1H) ; 3,1(m,1H) ; 1,8-1,1(m,8H) ;1,0(s,3H) ; 0,9(s,3H) |
| *IV.7* | 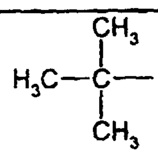 | Cl | |
| *IV.8* | 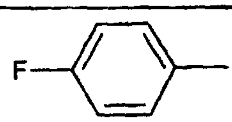 | Cl | 139 |
| *IV.9* | 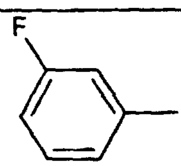 | Cl | |

**TABLEAU 2 (suite 1)**

| COMPOSE | $R_1$ | X | F ; °C ou $^1H$ RMN |
|---|---|---|---|
| *IV.10* | 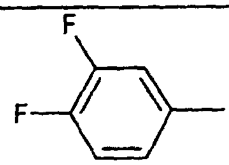 | Cl | |
| *IV.11* | 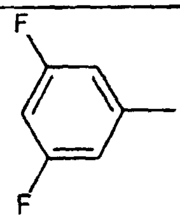 | Cl | |
| *IV.12* | 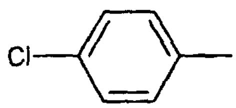 | Cl | |
| *IV.13* | 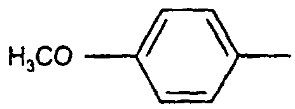 | Cl | 10,1(d,1H) ; 8,0(s,1H) ; 7,9(d,1H) ; 7,6-7,3(m,3H) ; 7,1(m,2H) ; 7,0(d,1H);3,8(s,3H) |
| *IV.14* | 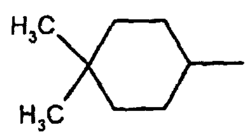 | Cl | 10(d,1H) ; 7,9(s,1H) ; 7,8(d,1H) ; 7,5(d,1H) ; 7,0(d,1H) ; 4,8(m,1H) ; 1,7-1,1(m,8H) ; 0,95(s,3H) ; 0,9(s,3H) |

**TABLEAU 3**

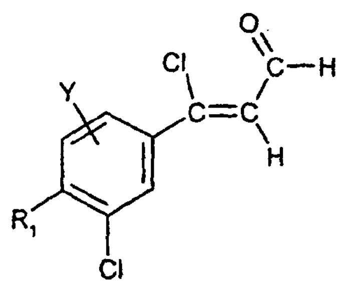

(IV) avec X = Cl

| COMPOSE | $R_1$ | Y | F ; °C ou $^1H$ RMN |
|---|---|---|---|
| *IV.15* | 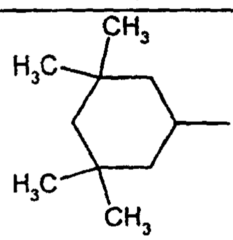 | 5-Cl | 10,1 (d,1H) ; 8,0 (s,1H) ; 7,9 (s,1H) ; 7,1 (d,1H) ; 3,9 (m,1H) ; 2,1 (m,2H) ; 1,3 (m,4H) ; 1,1 (s,6H) ; 0,9 (s,6H) |

**TABLEAU 3 (suite 1)**

| COMPOSE | $R_1$ | Y | F ; °C ou $^1$H RMN |
|---|---|---|---|
| *IV.16* | 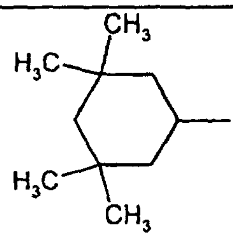 | 6-Cl | 10,0 (d,1H); 7,8-7,4 (m,2H) ; 6,6 (d,1H) ; 3,2 (m,1H) ; 1,6-1,2 (m,6H) ; 1,0 (s,6H) ; 0,9 (s,6H) |
| *IV.17* | 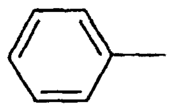 | 5-Cl | 108 |

**PREPARATION 16**

**3-Chloro-4-(3,3,5,5-tétraméthylcyclohexyl)phényléthyne,** ***composé II.1.***

(II.1) : $R_1$ = 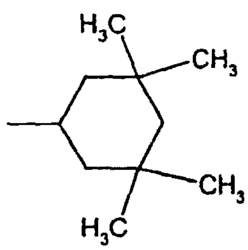 ; X = 3-Cl ; Y = H

Sous atmosphère inerte et sous forte agitation on dissout 5,3 g d'hydroxyde de sodium dans 150 ml d'eau. On ajoute 80 ml de 1,4-dioxane et chauffe à reflux. On additionne rapidement 15 g de 3-chloro-3-[3-chloro-4-(3,3,5,5-tétraméthylcyclohexyl)phényl] propénal (*composé IV.1)* en solution dans 130 ml de 1,4-dioxane et maintient le mélange réactionnel à reflux pendant 1 heure. Après retour à température ambiante on verse le mélange réactionnel sur un grand volume de dichlorométhane. On décante, sèche la phase organique sur sulfate de magnésium et évapore les solvants sous pression réduite. On purifie par chromatographie sur une colonne de gel de silice en éluant avec du cyclohexane ; rdt = 80%.

$^1$H RMN : 7,5 (s,1H) ; 7,3 (m,2H) ; 4,2 (s,1H) ; 3,2 (m,1H) ; 1,4 (m,2H) ; 1,2 (m,4H) ; 1,0 (s,6H) ; 0,9 (s,6H).

De la même manière, on prépare les *composés II.2* à *II.15* présentés dans les TABLEAUX 4 et 5 ci-après :

## TABLEAU 4

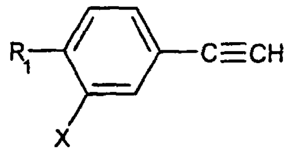

(II) avec Y = H

| COMPOSE | $R_1$ | X | F ; °C ou $^1$H RMN |
|---|---|---|---|
| *II.2* | 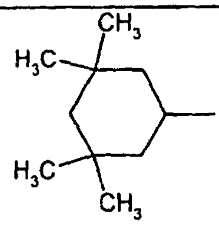 | H | 7,3 (d,2H) ; 7,2 (d,2H) ; 4,1 (s,1H) ; 2,9 (m,1H) ; 1,5-1,1 (m,6H) ; 1,0 (s,6H) ; 0,9 (s,6H) |
| *II.3* | 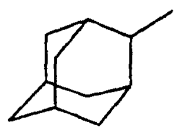 | H | |
| *II.4* | 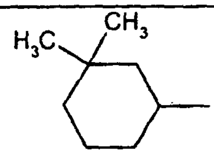 | Cl | 7,4 (s,1H) ; 7,3 (d,1H) ; 7,2 (d,1H) ; 4,0 (s,1H) ; 3,0 (m,1H) ; 1,7-1,0 (m,8H) ; 0,9 (s,3H) ; 0,8 (s,3H) |
| *II.5* | 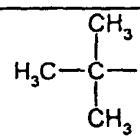 | Cl | 7,4 (m,3H) ; 4,2 (s,1H) ; 1,3 (s,9H) |
| *II.6* | 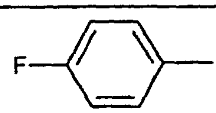 | Cl | 7,6 (s,1H) ; 7,4 (m,6H) ; 4,3 (s,1H) |
| *II.7* | 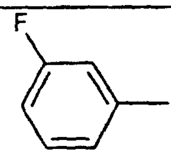 | Cl | 7,7 (s,1H) ; 7,5 (m,3H) ; 7,3 (m,3H) ; 4,3 (s,1H) |
| *II.8* | 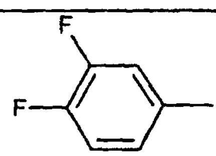 | Cl | 7,7 (s,1H) ; 7,5 (m,4H) ; 7,3 (m,1H) ; 4,3 (s,1H) |
| *II.9* | 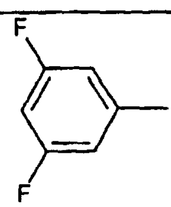 | Cl | |
| *II.10* | 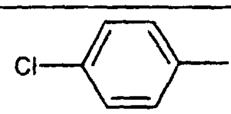 | Cl | 78 |

## TABLEAU 4 (suite 1)

| COMPOSE | $R_1$ | X | F ; °C ou $^1$H RMN |
|---|---|---|---|
| *II.11* | 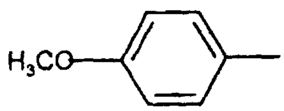 | Cl | 7,6 (s,1H) ; 7,4 (d,1H) ; 7,3 (m,3H) ; 7,0 (d,2H) ; 4,3 (s,1H) ; 3,8 (s,3H) |
| *II.12* | 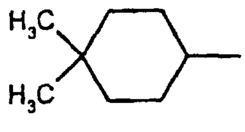 | Cl | 7,5(s,1H) ; 7,4(m,2H) ; 4,2(s,1H) ; 2,8(m,1H) ; 1,7-1,2(m,8H) ; 0,95(s,3H) ; 0,9(s,3H) |

## TABLEAU 5

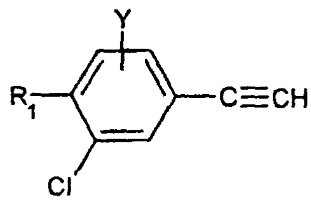 (II) avec X = Cl

| COMPOSE | $R_1$ | Y | F ; °C ou $^1$H RMN |
|---|---|---|---|
| *II.13* | 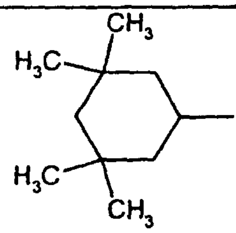 | 5-Cl | 7,6 (s,1H); 7,5 (s,1H) ; 4,4 (s,1H) ; 3,9 (m,1H) ; 2,0 (t,2H) ;1,2 (m,4H); 1,1 (s,6H) ; 0,9 (s,6H) |
| *II.14* | 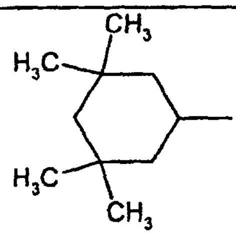 | 6-Cl | 7,6 (s,1H) ; 7,4 (s,1H) ; 4,6 (s,1H) ; 3,2 (m,1H) ; 1,5-1,1 (m,6H) ; 1,0 (s,6H) ; 0,9 (s,6H) |
| *II.15* | 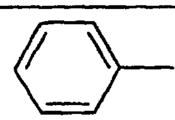 | 5-Cl | 7,7 (s,2H) ; 7,4 (m,3H) ; 7,2 (d,2H) ; 4,5 (s,1H) |

**PREPARATION 17**

**Acide 3,5-difluorobenzèneboronique, *composé 2.1***

A -78°C, à 20 g de 1-bromo-3,5-fluorobenzène dans 300 ml d'éther diéthylique, on additionne 91,5 ml de *tert*-butyllithium. On agite le mélange réactionnel pendant 1 heure à -78°C puis additionne 14,2 ml de triméthylborate. On agite le mélange réactionnel pendant 1 heure à -78°C
puis pendant 12 heures à température ambiante. On additionne 200 ml d'une solution aqueuse 1 N d'acide chlorhydrique. On extrait à l'éther diéthylique, lave la phase organique avec une solution saturée en hydrogénocarbonate de sodium, la sèche sur sulfate de magnésium et évapore les solvants sous pression réduite. On reprend le résidu au cyclohexane et isole le précipité obtenu par filtration.
$^1$H RMN :7,4 (m,3H) ; 7,2 (m,2H).

**PREPARATION 18**

**4-Bromo-3-chloroacétophénone, *composé Va.3***

(Va.3) ; X = 3-Cl ; Y = H ; Z = Br

A 0°C, à 133,34 g de chlorure d'aluminium dans 600 ml de dichlorométhane, on additionne goutte à goutte une solution de 100 g de 4-bromoacétophénone dans 250 ml de dichlorométhane. Après 2 heures d'agitation à 0°C, on fait barboter dans le milieu à 0°C 28,3 ml de chlore préalablement congelé (-75°C). On agite à température ambiante pendant 12 heures puis hydrolyse le mélange réactionnel. On décante, extrait au dichlorométhane, sèche les phases organiques sur sulfate de magnésium et évapore les solvants sous pression réduite. On recristallise le résidu obtenu dans l'hexane ; rdt = 57% ; F = 80°C.

**PREPARATION 19**

**3-Chloro-3-(4-bromo-3-chlorophényl)propénal, composé *IVa.1***

(IVa.1) : X = 3-Cl ; Y = H ; Z = Br

A une température comprise entre 3 et 6°C, on additionne sous forte agitation 15,08 ml de chlorure d'oxalyle à 16 ml de diméthylformamide dans 200 ml de dichlorométhane. Après retour à température ambiante, on agite pendant 30 minutes puis ajoute une solution de 13,4 g de 4-bromo-3-chloroacétophénone (composé *Va.3)* dans 40 ml de dichlorométhane. On agite le mélange réactionnel pendant 12 heures à température ambiante, hydrolyse le mélange réactionnel par ajout d'une solution de 18,9 g d'acétate de sodium dans 50 ml d'eau. Après 30 minutes d'agitation à température ambiante, on décante, extrait au dichlorométhane, sèche les phases organiques sur sulfate de magnésium et évapore les solvants sous pression réduite. Le résidu obtenu est recristallisé dans le cyclohexane ; rdt = 87% ; F = 134°C.

**PREPARATION 20**

**[3-(4-Bromo-3-chlorophényl)prop-2-ynyl]cyclohexyléthylamine, *composé Ia.2*.**

(Ia.2) : X = 3-Cl ; Y = H ; Z = Br ; 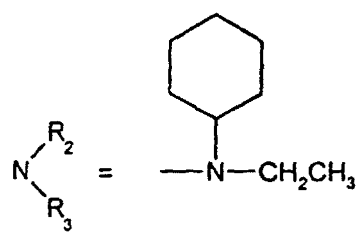

a) 1-Bromo-2-chloro-4-éthynylbenzène

Sous atmosphère inerte, on dissout 40 g d'hydroxyde de sodium dans 230 ml d'eau, on additionne 120 ml de 1,4-dioxane et chauffe le mélange réactionnel à 80°C. On additionne 17,5 g de 3-chloro-3-(4-bromo-3-chlorophényl)propénal en solution dans 400 ml de 1,4-dioxane et agite le mélange réactionnel pendant 30 minutes à 80°C. On laisse revenir le mélange réactionnel à température ambiante puis additionne 2300 ml de dichlorométhane. On décante, lave la phase organique à l'eau et la sèche sur sulfate de magnésium. Le composé en solution dans un mélange dichlorométhane/1,4-dioxane est engagé tel quel dans l'étape suivante.

b) [3-(4-Bromo-3-chlorophényl)prop-2-ynyl]cyclohexyléthylamine.

On ajoute une solution aqueuse à 36% de formaldéhyde sur 10,36 ml d'éthylcyclohexylamine dans 400 ml de 1,2-diméthoxyéthane. On additionne cette solution à la solution du composé obtenu précédemment en présence de 0,54 g de chlorure de cuivre II , dihydraté. On agite le mélange réactionnel pendant 4 heures à reflux puis laisse le mélange réactionnel revenir à température ambiante. On filtre, évapore les solvants sous pression réduite puis purifie le résidu obtenu par chromatographie sur une colonne de gel de silice en éluant avec un mélange dichlorométhane/éthanol, 99/1 (v/v). On reprend le composé obtenu à l'éther diéthylique et fait barboter de l'acide chlorhydrique. On filtre et sèche le précipité obtenu pour obtenir le composé sous forme de chlorhydrate.

$^1$H RMN : 7,7 (d,1H) ; 7,6 (s,1H) ; 7,2 (d,1H) ; 3,5 (s,2H) ; 2,6 (q,2H) ; 2,4 (m,1H) ; 1,8-1,1 (m,10H) ; 0,9 (t,3H).

**PREPARATION 21**

**2-Chloro-4-(4,4-diméthylcyclohexyl)phénol, composé IX.1**

a) 2-Chloro-4-(1-hydroxy-4,4-diméthylcyclohexyl)phénol

A 15,1 g de 4-bromo-2-chlorophénol dans 150 ml de tétrahydrofurane, on additionne à -78°C 100 ml d'une solution 1,6M de n-butyllithium dans l'hexane, agite le mélange réactionnel pendant 1 heure à -78°C. On additionne 10,1 g du 4,4-diméthylcyclohexanone (*composé 3.1*) et agite à nouveau le mélange réactionnel pendant 30 minutes à -78°C puis pendant 12 heures à température ambiante. Le mélange réactionnel est hydrolysé par une solution d'acide chlorhydrique 1N et extrait avec de l'acétate d'éthyle. La phase organique est séchée sur sulfate de magnésium et les solvants évaporés sous pression réduite. Le solide obtenu est purifié par chromatographie sur une colonne de gel de silice en éluant avec un mélange cyclohexane/acétate d'éthyle variant de 98/2 à 90/10 (v/v). On obtient 11,8 g de solide. $^1$H RMN : 7,4(s,1H) ; 7,2(d,2H) ; 6,9(d,2H) ; 4,5(s,1H) ; 1,9-1.1(m,8H) ; 0,9(s,6H)

b) A 11,8 g du 2-chloro-4-(1-hydroxy-4,4-diméthylcyclohexyl)phénol dans 200 ml d'acide acétique, on additionne 50 ml d'une solution aqueuse 'd'acide iodhydrique à 57%. Le mélange réactionnel est chauffé à reflux pendant 3 heures, les solvants évaporés sous pression réduite. On ajoute une solution aqueuse à 40% d'hydroxyde de sodium, une solution aqueuse de carbonate de sodium, puis une solution aqueuse d'hydrogénosulfate de sodium et extrait avec de l'éther diéthylique. La phase organique est séchée sur sulfate de magnésium et les solvants évaporés sous pression réduite. On purifie le composé obtenu par chromatographie sur colonne de gel de silice en éluant avec un mélange cyclohexane/acétate d'éthyle 95/5 (v/v).
$^1$H RMN : 9,8(s,1H) ; 7,1(s,1H) ; 7(d,1H) ; 6,9(d,1H) ; 1,9(m,1H) ; 1,6-1,2(m,8H) ; 0,9(s,6H)

Selon le même mode opératoire, on prépare les *composés IX.2 à IX.4* :
**4-(Adamantan-2-yl)-3,5-dichlorophénol, composé IX.2** obtenu à partir du composé IXa.1 et de l'adamantan-2-one
$^1$H RMN : 10,1(s,1H) ; 6,8(s,2H) ; 3,4(s,1H); 2,4(s,2H) ; 2,3-1,4(m,12H)
**4-(Adamantan-2-yl)phénol, composé IX.3**
$^1$H RMN : 9,1(s,1H) ; 7,1(d,2H) ; 6,7(d,2H) ; 2,8(s,1H) ; 2,4(s,2H) ; 1,9-1,4(m,12H) **4-(Adamantan-2-yl)-3-chlorophénol, composé IX.4**
$^1$H RMN : 9,8(s,1H) ; 7,1(s,1H) ; 7,0(d,1H) ; 6,9(d,1H) ; 2,8(s,1H) ; 2,3(m,2H) ; 1,9(m,5H) ; 1,7(m,5H) ; 1,5(m,2H)

**PREPARATION 22**

**4-(Tétrahydropyran-4-yl)phénol, composé IX.5**

a) 4-(3,6-Dihydropyran-4-yl)phénol

A 12,7 g de 4-bromophénol dans 150 ml de tétrahydrofurane, on additionne à -40°C sur le mélange réactionnel 100 ml de butyllithium à 1,6M dans l'hexane, puis 8,1 g de 4-tétrahydropyranone. On laisse le mélange réactionnel pendant 18 heures à température ambiante, puis hydrolyse à l'acide chlorhydrique 1N. On extrait plusieurs fois à l'éther diéthylique, sèche la phase organique sur sulfate de magnésium et évapore les solvants sous pression réduite. Le solide obtenu est purifié par chromatographie sur une colonne de gel de silice en éluant avec un mélange cyclohexanelacétate d'éthyle variant de 90/10 à 80/20 (v/v). $^1$H RMN : 9,4(s,1H) ; 7,2(d,1H) ; 6,7(d,1H) ; 6,0(t,1H) ; 4,1(d,2H) ; 3,7(t,2H) ; 2,4(t,2H)

b) 5,5g de 4-(3,6-dihydropyran-4-yl)phénol sont hydrogénés en présence de 550 mg de palladium sur charbon à 10% dans 100 ml de méthanol, pendant 3 heures. On filtre puis évapore les solvants sous pression réduite.
$^1$H RMN : 9,1(s,1H) ; 7(d,2H) ; 6,6(d,2H) ; 3,9(m,2H) ; 3,4(m,2H) ; 2,6(m,1H) ; 1,6(m,4H)

De la même manière, on prépare le composé suivant :
**4-(4,4-Diméthylcyclohexyl)phénol, composé IX.6**
$^1$H RMN : 9(s,1H) ; 7(d,2H) ; 6,7(d,2H) ; 2,2(m,1H) ; 1,6-1,2(m,8H) ; 0,9(s,6H)

**PREPARATION 23**

**4-(Adamantan-2-yl)-3,5-difuorophénol, composé IX.7**

a) 2-(2,6-difluoro-4-méthoxyphényl)adamantan-2-ol
Obtenu à partir du 4-bromo-3,5-difluorophénylméthyléther en présence de un équivalent de n-butyllithium selon le mode opératoire décrit à la PREPARATION 22 a).
b) 19 g du produit obtenu à l'étape précédente, 200 ml d'acide iodhydrique et 200 ml d'acide acétique sont agités une nuit à la température de reflux. Après retour à température ambiante, le mélange réactionnel est versé sur un mélange glace pilée /$NaHSO_3$. Après neutralisation par une solution de soude 1N, on extrait au dichlorométhane. La phase organique est séchée sur sulfate de magnésium puis les solvants sont évaporés sous pression réduite.

**PREPARATION 24**

**2-Chloro-4-(4,4-diméthylcyclohexyl)phényltrifluorométhanesulfonate, composé III.1**

A 9,7 g du 2-chloro-4-(4,4-diméthylcyclohexyl)phénol *(composé IX.1)* dans 60 ml de pyridine, on additionne à 5°C 8,2 ml d'anhydride triflique et laisse le mélange réactionnel pendant 30 minutes à 0°C, puis on agite le mélange réactionnel à température ambiante pendant 12 heures. Le mélange réactionnel est hydrolysé puis extrait au dichlorométhane. La phase organique est séchée sur sulfate de magnésium et les solvants évaporés sous pression réduite. Le résidu obtenu est repris avec du toluène puis les solvants sont évaporés sous pression réduite. Le résidu obtenu est purifié par chromatographie sur une colonne de gel de silice en éluant avec un mélange cyclohexane /acétate d'éthyle variant de 100/0 à 99/1 (v/v). On obtient 15 g du composé.
$^1$H RMN : 7,7(s,1H) ; 7,5(d,1H) ; 7,4(d,1H) ; 2,5(m,1H) ; 1,6-1,2(m,8H) ; 0,92(s,3H) ; 0,86(s,3H).
Selon le même mode opératoire, on prépare les composés III.2 à III.7 :

**4-(Adamantan-2-yl)-3,5-dichlorophényltrifluorométhanesulfonate, composé III.2**
$^1$H RMN : 7,7(d,1H) ; 7,6(d,1H) ; 3,6(m,1H) ; 3,0-1,0(m,14H)
**4-(Adamantan-2-yl)phényltrifluorométhanesulfonate, composé III.3**
$^1$H RMN : 7,5(d,2H) ; 7,4(d,2H) ; 3,0(s,1H) ; 2,4(s,2H) ; 1,9(m,5H) ; 1,8-1,5(m,7H)
**4-(Adamantan-2-yl)-3-chlorophényltrifluorométhanesulfonate, composé III.4**
$^1$H RMN : 7,6-7,4(m,3H) ; 3,0(s,1H) ; 2,4(m,2H) ; 1,9(m,5H) ; 1,8-1,4(m,7H)
**4-(Adamantan-1-yl)phényltrifluorométhanesulfonate, composé III.5**
$^1$H RMN : 7,5(d,2H) ; 7,3(d,2H) ; 2,1(m,3H) ; 1,8(m,6H) ; 1,7(m,6H)
**4-(Tétrahydropyran-4-yl)phényltrifluorométhanesulfonate, composé III.6**
$^1$H RMN : 7,4(s,4H) ; 3,9(m,2H) ; 3,4(m,2H) ; 2.8(m,1H) ; 1,7(m,4H)
**4-(4,4-Diméthylcyclohexyl)phényltrifluorométhanesulfonate, composé III.7**
$^1$H RMN : 7,4-7,3(m,4H) ; 2,6(m,1H) ; 1,6-1,2(m,8H) ; 0,93(s,3H) ; 0,90(s,3H)

Les composés des EXEMPLES ci-après sont, sauf mention contraire, de formule (I) avec : n = 1 et $-NR_2R_3$ =

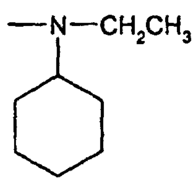

## EXEMPLE 1

**Chlorhydrate de [3-(4-adamantan-2-ylphényl)prop-2-ynyl]cyclohexyléthylamine.**

(I) : $R_1$ = 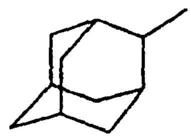 ; X = Y = H ; A = $-C\equiv C-$

A 11,2 ml de cyclohexyléthylamine dans 100 ml de 1,2-diméthoxyéthane, on additionne 8,6 ml de formaldéhyde à 36% et on maintient l'agitation à température ambiante pendant 2 heures. On additionne cette solution à un mélange de 16 g de 2-(4-éthynylphényl)adamantane (*composé II.3 ),* 0,58 g de chlorure de cuivre II, dihydraté dans 400 ml de 1,2-diméthoxyéthane. On chauffe le mélange réactionnel à reflux pendant 2 heures puis évapore les solvants sous pression réduite. On reprend le composé obtenu avec de l'éther diéthylique et fait barboter de l'acide chlorhydrique, filtre et sèche le précipité obtenu ; F = 124°C (HCl, 0,5 $H_2O$).

De la même manière, on prépare les composés des EXEMPLES 2 à 12 présentés ci-après.

## EXEMPLE 2

**Chlorhydrate de {3-[4-(3,3,5,5-tétraméthylcyclohexyl)phényl]prop-2-ynyl}cyclo hexyléthylamine.**

(I) : $R_1$ = 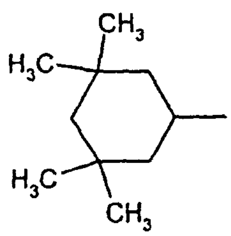 ; X = Y = H ; A = $-C\equiv C-$

F = 150°C (HCl, 0,1 $H_2O$)

## TABLEAU 6

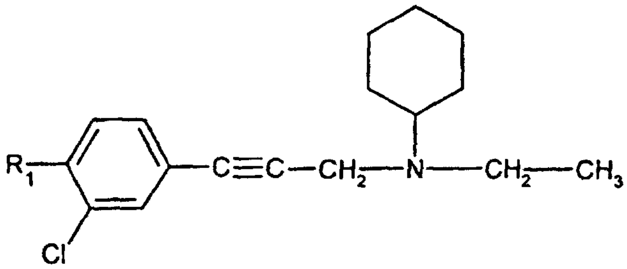

(I)

| EXEMPLE | $R_1$ | F ; °C (sel, hydrate) |
|---|---|---|
| 3 | 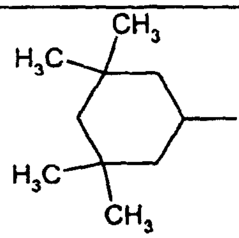 | 99<br>HCl |
| 4 | 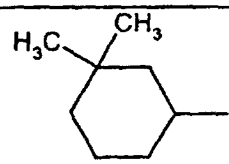 | 137<br>HCl |
| 5 | 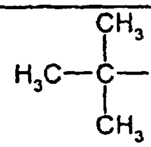 | 156<br>HCl<br>0,1 $H_2O$ |
| 6 | 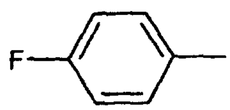 | 148<br>HCl<br>0,2 $H_2O$ |

## TABLEAU 6 (suite 1)

| EXEMPLE | $R_1$ | F ; °C (sel, hydrate) |
|---|---|---|
| 7 | 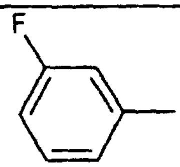 | 130<br>HCl<br>0,2 $H_2O$ |
| 8 | 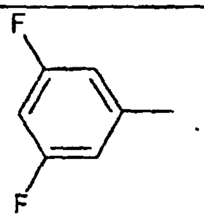 | 0,96(t,3H) ;<br>1,2-1,8(m,11H) ;<br>2,6(q,2H) ; 3,6(s,1H)<br>7,1-7,4(m,5H) ;<br>7,6(s,1H) |
| 9 | 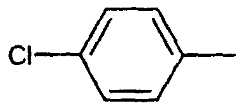 | 172<br>HCl |
| 10 | 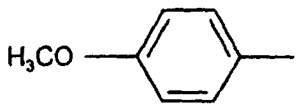 | 50 (pâteux)<br>HCl<br>0,7 $H_2O$ |
| 11 | 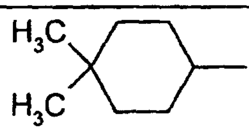 | (a)<br>HCl |

(a) $^1H$ RMN : 7,4(m,2H) ; 7,3(d,1H) ; 3,6(s,2H) ; 3,4(m,1H) ; 2,8(m,1H) ; 2,6(q,2H) ; 1,3-0,9(m,27H)

### EXEMPLE 12

**Chlorhydrate de [3-(2,6-dichlorobiphényl-4-yl)prop-2-ynyl]cyclohexyléthylamine.**

(I) : $R_1$ = 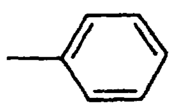 ; X = 3-Cl ; Y = 5-Cl ; A = $-C\equiv C-$

F = 205°C (HCl).

### EXEMPLE 13

Composé identique à celui de L'EXEMPLE 7 mais préparé différemment.

**Chlorhydrate de 3-(2-Chloro-3'-fluorobiphényl-4-yl)prop-2-ynyl]cyclohexyl éthylamine.**

(I) : $R_1$ = 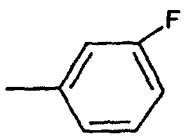 ; X = 3-Cl ; Y = H ; A = —C≡C—

Sous atmosphère inerte, on agite pendant 4 heures à reflux 3,4 g de 2-chloro-4-[3-(cyclohexyléthylamino) prop-1-ynyl]phényltrifluorométhanesulfonate (*composé Ia.1),* 1,23 g d'acide 3-fluorobenzèneboronique 2,2 g de carbonate de sodium dans 10,4 ml d'eau, 0,68 g de chlorure de lithium, 75 ml de toluène, 25 ml d'éthanol et 0,7 g de tétra (triphénylphosphine)palladium. On filtre, évapore les solvants sous pression réduite et purifie le résidu obtenu par chromatographie sur une colonne de gel de silice en éluant avec un mélange dichlorométhane/éthanol, 99/1 (v/v). On reprend le composé obtenu avec de l'éther diéthylique, fait barboter de l'acide chlorhydrique. On filtre, évapore les solvants sous pression réduite ; F = 130°C (HCl, 0,2 $H_2O$).

De la même manière, on prépare les composés des EXEMPLES 14 et 15 ci-après :

## TABLEAU 7

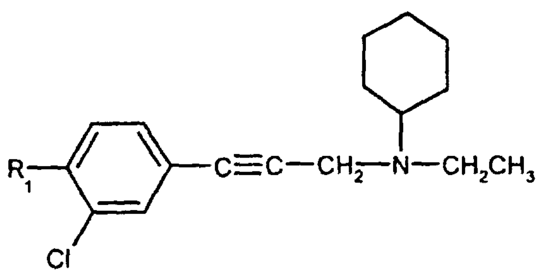 (I)

| EXEMPLE | $R_1$ | F ; °C (sel, hydrate) |
|---|---|---|
| 14 | 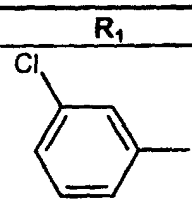 | 155<br>HCl |
| 15 | 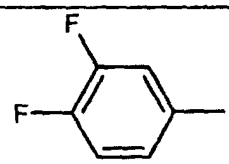 | 139<br>HCl<br>0,3 $H_2O$ |

**EXEMPLE 16**

**Chlorhydrate de [3-(4-adamantan-2-yl-3-chlorophényl)prop-2-ynyl]cyclohexyl éthylamine.**

(I) : $R_1$ = 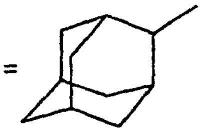 ; X = 3-Cl ; Y = H ; A = —C≡C—

a) **2-{2-chloro-4-[3-(cyclohexyléthylamino)prop-1-ynyl]phényl}adamantan-2-ol,**

On traite le chlorhydrate de la [3-(4-bromo-3-chlorophényl)propen-2-ynyl]cyclohexyl éthylamine avec une solution 1 N d'hydroxyde de sodium dans l'éther pour obtenir la base. A -75°C, à 17,5 g de [3-(4-bromo-3-chlorophényl)propen-2-ynyl]cyclohexyl éthylamine dans 200 ml d'éther diéthylique, on additionne 30,5 ml d'une solution de n-butyllithium à 15% dans l'hexane et maintient l'agitation à -75°C pendant 1 heure 30 minutes. Toujours à -75°C, on additionne 7,51 g d'amandatan-2-one dans 100 ml d'éther diéthylique puis agite le mélange réactionnel pendant 2 heures à -75°C.

On laisse le mélange réactionnel revenir à température ambiante et maintient l'agitation pendant 1 heure. On hydrolyse le mélange réactionnel, extrait à l'éther diéthylique sèche les phases organiques sur sulfate de magnésium et évapore les solvants sous pression réduite. Le résidu obtenu est purifié par chromatographie sur une colonne de gel de silice en éluant avec un mélange dichlorométhane/éthanol variant de 100/0 à 99/1 (v/v). Le composé obtenu est directement engagé dans l'étape suivante.

b) A 11,12 g du composé obtenu précédemment dans 50 ml d'acétonitrile et 25 ml de dichlorométhane, on additionne 9,78 g d'iodure de sodium puis 6,63 ml de chlorotriméthylsilane. On agite le mélange réactionnel pendant 2 heures à 30°C puis additionne 25 ml d'acétonitrile, 5,12 g de zinc en poudre et 2,99 ml d'acide acétique. On chauffe le mélange reactionnel à 80°C pendant 3 heures , laisse revenir à température ambiante, filtre, lave à l'éther diéthylique, extrait au dichlorométhane puis évapore les solvants sous pression réduite. Le résidu obtenu est purifié par chromatographie sur une colonne de gel de silice en éluant avec un mélange toluène/éthanol 97/3 (v/v) puis avec un mélange cyclohexane/acétate d'éthyle 92,5/7,5 (v/v). On reprend le composé obtenu avec de l'éther diéthylique, et prépare le chlorhydrate en faisant barboter de l'acide chlorhydrique, filtre et sèche le précipité obtenu ; F = 110°C (HCl, 0,3 $H_2O$).

**EXEMPLE 17**

**Chlorhydrate de {3-[4-(4,4-diméthylcyclohexyl)-2-chlorophényl]prop-2-ynyl} cyclohexyléthylamine**

(I) : $R_1$ = 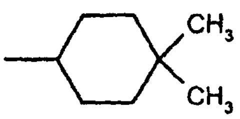 ; X = 2-Cl ; Y = H ; A = —C≡C—

Sous atmosphère inerte, on additionne 1,42 g de dichlorodi(triphénylphosphine) palladium à 8,03 g de cyclohexyléthylprop-2-ynylamine (*composé 4.1),* 15 g de [4-(4,4-diméthylcyclohexyl)-2-chlorophényl]trifluorométhanesulfonate (*composé III.1)*, 0,19 g d'iodure de cuivre, 3,4 g de chlorure de lithium dans 200 ml de triéthylamine et 100 ml de pyridine. Le mélange réactionnel est chauffé à reflux pendant 12 heures. On évapore les solvants sous pression réduite et purifie le résidu obtenu par chromatographie sur une colonne de gel de silice en éluant avec un mélange cyclohexane/acétate d'éthyle variant de 95/5 à 90/10 (v/v). On reprend le résidu obtenu avec de l'éther diéthylique. Le chlorhydrate est séparé par filtration puis on fait barboter de l'acide chlorhydrique. Le résidu obtenu est recristallisé dans l'acétate d'éthyle.

$^1$H RMN : 11(s,11H) ; 7,6-7,4(m,2H) ; 7,3(d,1H) ; 4,3(s,2H) ; 3,2(m,2H) ; 1,5(m,1H) ; 2,2-1,1(m,22H) ; 0,9(d,6H).

De la même manière, on prépare les composés des EXEMPLES 18 à 28 ci-après :

**EXEMPLE 18**

**Chlorhydrate de [4-(4-adamantan-2-yl-2-chlorophényl)but-3-ynyl]cyclohexyl éthylamine**

(I) : $R_1$ = 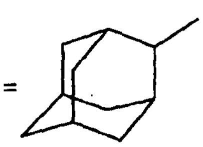 ; X = 2-Cl ; Y = H ; A = —C≡C— ; n = 2

$^1$H RMN : 7,5(d,1H) ; 7,4(s,1H) ; 7,3(d,1H) ; 3,4-3,2(m,4H) ; 3,1(m,2H) ; 3,0(s,1H) ; 2,4(s,2H) ; 2,0-2,1(m,26H).

## TABLEAU 8

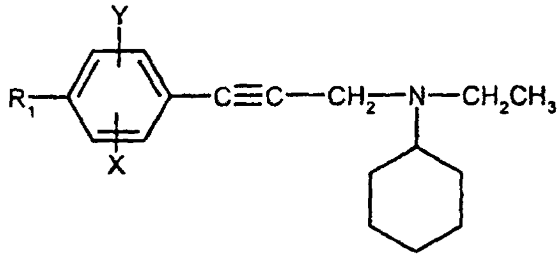

(I)

| EXEMPLE | $R_1$ | X | Y | F ; °C ou $^1H$ RMN (sel, hydrate) |
|---|---|---|---|---|
| 19 | 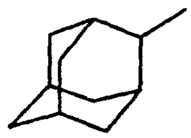 | 3-Cl | 5-Cl | 7,5(d,1H) ; 7,2(d,1H) ; 4,3(s,2H) ; 3,3(m,3H) ; 2,6-1,1(m,28H) HCl |
| 20 | 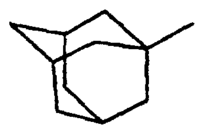 | H | H | 186 HCl 0,8 $H_2O$ |
| 21 | 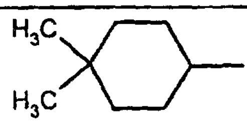 | H | H | 134 |
| 22 | 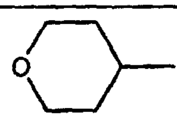 | H | H | 152 HCl |

**TABLEAU 8 (suite 1)**

| EXEMPLE | $R_1$ | X | Y | F ; °C ou $^1$H RMN (sel, hydrate) |
|---|---|---|---|---|
| 23[a] | 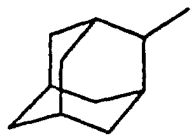 | 3-Cl | 6-Cl | 196 HCl |
| 24 | 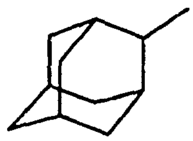 | 3-F | 5-F | 132 HCl |
| 25 | 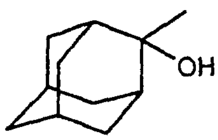 | 3-Cl | 5-Cl | 210 HCl |

[a] préparé selon le même schéma de synthèse que l'EXEMPLE 17 en utilisant comme produit de départ le 4-bromo-3-méthoxyphénol (J. Am. Chem. Soc. 1926, *48*, 3129)

**TABLEAU 9**

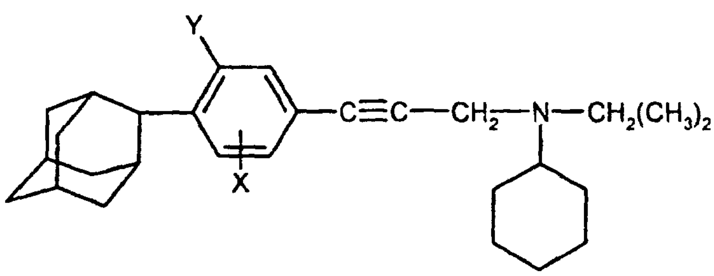 (I)

| EXEMPLE | X | Y | sel | $^1$H RMN |
|---|---|---|---|---|
| 26 | H | H | HCl | 10,3(s,1H) ; 7,4(m,4H) ; 4,3(s,2H) ; 3,8(m,1H) ; 2,4(s,2H) ; 2,1-1,1(m,30H) |
| 27 | 2-Cl | H | HCl | 10,4(s,1H) ; 7,6(d,1H) ; 7,5(s,1H) ; 7,4(d,1H) ; 4,4(s,2H) ; 3,8(m,1H) ; 3,4(m,1H) ; 2,9(s,1H) ; 2,4(s,2H) ; 2,1-1,2(m,28H) |
| 28 | 3-Cl | Cl | HCl | 10,3(s,1H) ; 7,6(s,2H) ; 4,3(s,2H) ; 3,5-1,0(m,32H) |

**EXEMPLE 29**

**Chlorhydrate de {(Z)-3-[3-chloro-4-(3,3,5,5-tétraméthylcyclohexyl)phényl]propèn-2-yl}cyclohexyléthylamine.**

(I) : $R_1$ = 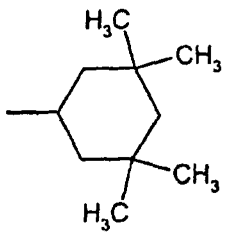 ; X = 3-Cl ; Y = H ; A = 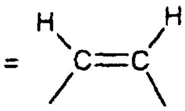

Sous atmosphère inerte et à pression atmosphérique, on hydrogène 3 g du composé de l'EXEMPLE 3 dans 50 ml d'éther de pétrole en présence de 3 ml de cyclohexène et 0,3 g de palladium sur carbonate de calcium empoisonné par 3,5% de plomb (catalyseur de Lindlar). On filtre sur célite, évapore les solvants et purifie le résidu obtenu par chromatographie sur une colonne de gel de silice en éluant avec un mélange dichlorométhane/éthanol, 95/5 (v/v). On reprend le résidu huileux obtenu avec du éther diéthylique et fait barboter de l'acide chlorhydrique. On filtre et sèche le précipité sous pression réduite. Le composé de l'EXEMPLE 29 est isolé avec un rendement de 83% ; F = 158°C (HCl, 0,1 $H_2O$).

De la même manière, on prépare les composés des EXEMPLES 30 à 54 présentés ci-après :

**TABLEAU 10**

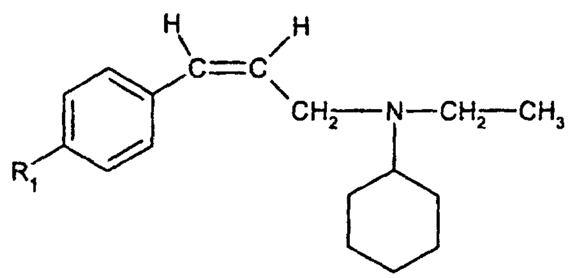 (I)

| EXEMPLE | $R_1$ | F ; °C (sel, hydrate) |
|---|---|---|
| 30 | 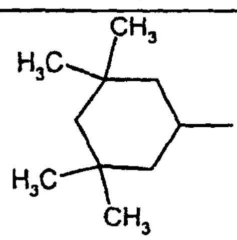 | 170<br>HCl |
| 31 | 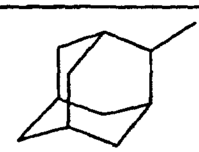 | 182<br>HCl |
| 32 | 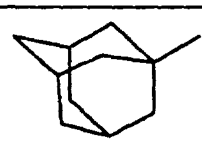 | 138<br>HCl<br>0,3 $H_2O$ |

TABLEAU 10 (suite 1)

| EXEMPLE | $R_1$ | F ; °C (sel, hydrate) |
|---|---|---|
| 33 | 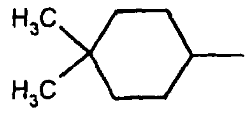 | 152 |
| 34 | 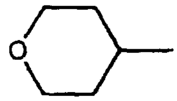 | 162<br>HCl |

## TABLEAU 11

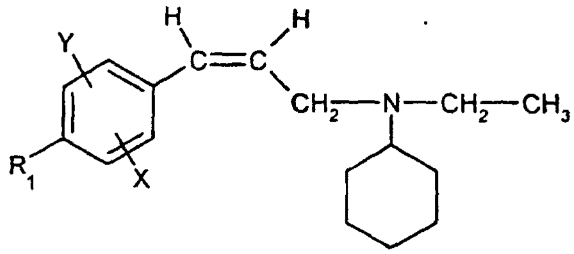

(I)

| EXEMPLE | $R_1$ | X | Y | F ; °C (sel, hydrate) |
|---|---|---|---|---|
| 35 | 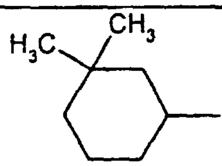 | 3-Cl | H | 155<br>HCl |
| 36 | 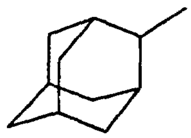 | 3-Cl | H | 114<br>HCl<br>0,5 $H_2O$ |
| 37 | 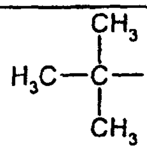 | 3-Cl | H | 144<br>HCl<br>0,3 $H_2O$ |
| 38 | 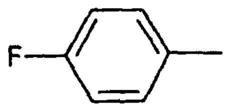 | 3-Cl | H | 105<br>HCl<br>1,1 $H_2O$ |
| 39 | 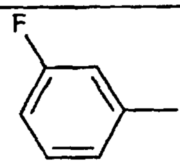 | 3-Cl | H | 108<br>HCl<br>0,6 $H_2O$ |
| 40 | 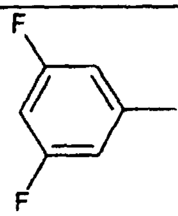 | 3-Cl | H | 138<br>HCl |

**TABLEAU 11 (suite 1)**

| EXEMPLE | $R_1$ | X | Y | F ; °C (sel, hydrate) |
|---|---|---|---|---|
| 41 | 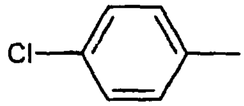 | 3-Cl | H | 160<br>HCl |
| 42 | 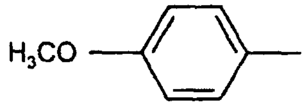 | 3-Cl | H | 70<br>HCl<br>0,7 $H_2O$ |
| 43 | 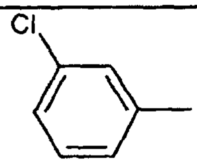 | 3-Cl | H | 102<br>HCl<br>0,4 $H_2O$ |
| 44[(a)] | 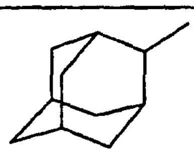 | 3-Cl | 5-Cl | 188<br>HCl |
| 45[(b)] | 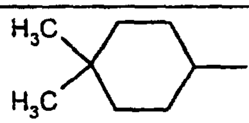 | 3-Cl | H | 161<br>HCl |
| 46 | 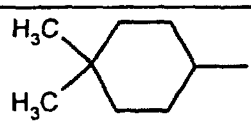 | 2-Cl | H | 195<br>HCl |

[(a)] à partir de la base correspondante, on prépare les sels de fumarate comme suit : 1g de base est solubilisée dans 50 ml d'isopropanol. 0,26 g d'acide fumarique sont également solubilisés à 50°C dans 100 ml d'isopropanol. La solution contenant le produit de départ est versée sur la solution tiède d'acide fumarique. Le mélange réactionnel est agité 15 minutes à température ambiante puis les solvants sont évaporés sous pression réduite. Les cristaux obtenus sont lavés à l'éther éthylique puis recristallisés dans l'acétonitrile ; F = 158°C (fumarate). De la même manière, on prépare le maléate : F =, 166°C (maléate)

[(b)] à partir de la base correspondante, on prépare le fumarate ; F = 104°C (fumarate)

**TABLEAU 12**

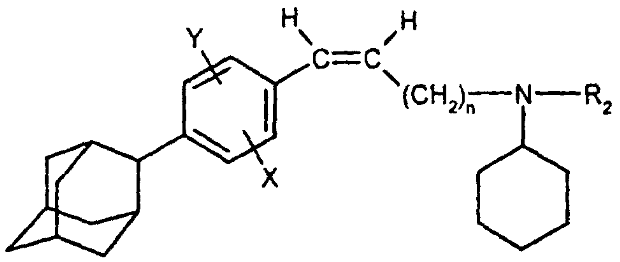

(I)

| EXEMPLE | X | Y | n | $R_2$ | F ; °C (sel, hydrate) |
|---|---|---|---|---|---|
| 47 | H | H | 1 | $-CH(CH_3)_2$ | (a) HCl |
| 48 | 3-Cl | H | 1 | $-CH(CH_3)_2$ | HCl 0,75 $H_2O$ |
| 49 | 3-Cl | 5-Cl | 1 | $-CH(CH_3)_2$ | 226 HCl |
| 50 | 2-Cl | H | 1 | $-CH(CH_3)_2$ | 162 HCl ; $H_2O$ |
| 51[(b)] | 3-Cl | 5-Cl | 1 | $-CH_3$ | 204 HCl |
| 52 | 3-Cl | 5-Cl | 2 | $-CH_2CH_3$ | 90 HCl ; 0,2 $H_2O$ |

(a) masse $ES^+$ : 392,4 ($MH^+$) ; 251,3 et 135,3

(b) préparé selon le même schéma de synthèse que l'EXEMPLE 44 en utilisant comme produit de départ le composé 4.2.

**EXEMPLE 53**

**Chlorhydrate de[(Z)-3-(2,6-dichlorobiphényl-4-yl)propèn-2-yl]cyclohexyléthylamine.**

(I) : $R_1$ = 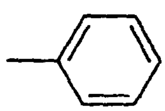 ; X = 3-Cl ; Y = 6-Cl ; A = 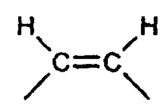

F = 120°C (HCl).

**EXEMPLE 54**

**Chlorhydrate de [(Z)-4-(4-adamantan-2-yl-3-chlorophényl)butèn-3-yl]cyclohexyl éthylamine**

(I) : $R_1$ = 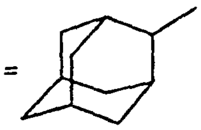 ; X = 3-Cl ; Y = H ; A = 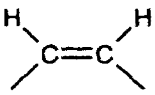 ; n = 2

F = 178°C (HCl).

Les composés du TABLEAU 13 ci-après, sont préparés selon le même schéma de synthèse que pour l'EXEMPLE 44 :

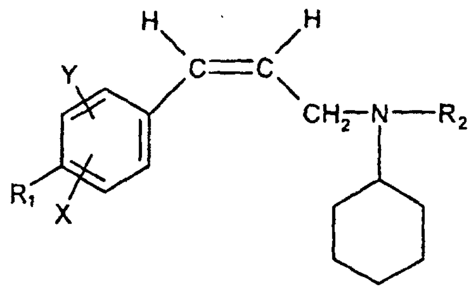

(I)

## TABLEAU 13

| EXEMPLE | X | Y | $R_1$ | $R_2$ | F ; °C sel, hydrate |
|---|---|---|---|---|---|
| 55 | 3-F | 5-F | 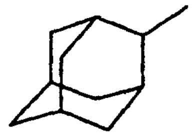 | $-C_2H_5$ | 182 HCl |
| 56[(a)] | $3-OCH_3$ | H | 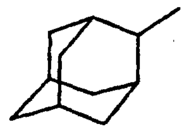 | $-C_2H_5$ | gommeux $HOC(O)CF_3$ |
| 57 | 3-Cl | 5-Cl | 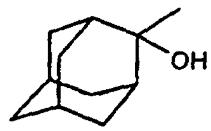 | $-C_2H_5$ | 210 HCl 0,2 $H_2O$ |
| 58 | 3-Cl | 6-Cl | 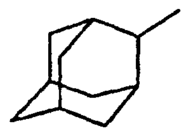 | $-C_2H_5$ | 165 HCl |
| 59 | 3-Cl | -H | 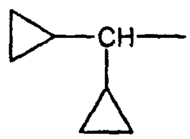 | $-C_2H_5$ | 140 HCl |
| 60[(b)] | 2-Cl | 6-Cl | 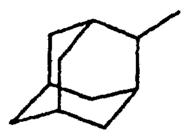 | $-C_2H_5$ | 174 HCl |
| 61 | -H | -H | 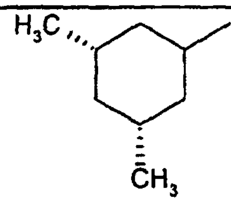 | $-C_2H_5$ | 142 HCl |

**TABLEAU 13 (suite 1)**

| EXEMPLE | X | Y | $R_1$ | $R_2$ | F ; °C sel, hydrate |
|---|---|---|---|---|---|
| 62 | 2-Cl | -H | 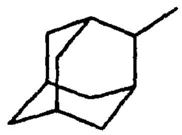 | $-C_2H_5$ | 208 HCl |
| 63 | 3-Cl | 5-Cl | 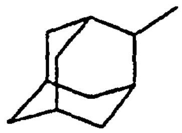 | -H | 152 HCl |

(a) en utilisant comme produit de départ le 4-bromo-3-méthoxyphénol (J. Am. Chem. Soc. 1926, *48*, 3129)

(b) en utilisant comme produit de départ le 4-bromo-2,6-dichlorophénol (J. Am. Chem. Soc 1933, *55*,2125-2126)

**EXEMPLE 64**

**Chlorhydrate de {(E)-3-[3-chloro-4-(3,3,5,5-tétraméthylcyclohexyl)phényl]propèn-2-yl}cyclohexyléthylamine.**

(I) : $R_1$ = 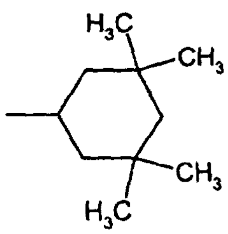 ; X = 3-Cl ; Y = H ; A = 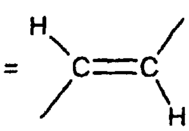

Sous atmosphère inerte, à une solution de 4 g du composé de l'EXEMPLE 4 dans 40 ml de toluène, on additionne goutte à goutte 24,3 ml d'une solution 1 M d'hydrure de diisobutylaluminium (DIBALH) dans le toluène. On agite le mélange réactionnel à 40°C pendant 1 heure puis verse le mélange réactionnel sur un mélange eau/glace et additionne de l'hydroxyde de sodium jusqu'à obtenir un pH égal à 7. On extrait au dichlorométhane, décante, sèche la phase organique sur sulfate de magnésium et évapore les solvants sous pression réduite. On reprend le résidu avec du éther diéthylique, fait barboter de l'acide chlorhydrique. On filtre et sèche le précipité obtenu ; F = 169°C (HCl, 0,2 $H_2O$).

Les composés des l'EXEMPLES 65 à 67 ci-après sont préparés selon le mode opératoire décrit pour l'EXEMPLE 64.

**EXEMPLE 65**

**Chlorhydrate de {(E)-3-[4-(3,3,5,5-tétraméthylcyclohexyl)phényl]propèn-2-yl} cyclohexyléthylamine.**

(I) : $R_1$ = 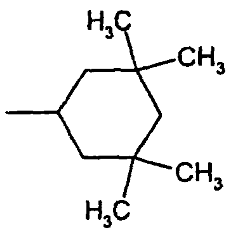 ; X = H ; Y = H ; A = 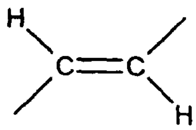

F = 200°C (HCl).

**EXEMPLE 66**

**Chlorhydrate de {(E)-3-[4-(2-adamantyl)phényl]propén-2-yl)cyclohexyléthylamine**

(I) : $R_1$ = 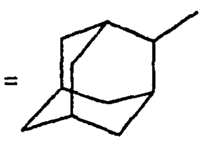 ; X = Y = H ; A = 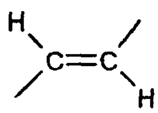

F = 200°C (HCl)

**EXEMPLE 67**

**Chlorhydrate de {(E)-3-[4-(2-Adamantyl)-3,5-dichlorophényl]propén-2-yl} cyclohexyléthylamine**

(I) : $R_1$ = 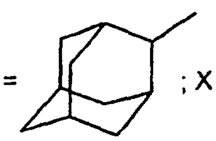 ; X = 3-Cl ; Y = 5-Cl ; A = 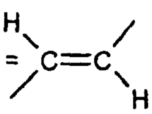

F = 224°C (HCl)

**EXEMPLE 68**

**Chlorhydrate de {3-[3-chloro-4-(3,3,5,5-tétraméthylcyclohexyl)phényl]propyl} cyclohexyléthylamine.**

(I) : $R_1$ = 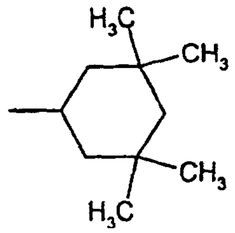 ; X = 3-Cl ; Y = H ; A = $-CH_2-CH_2-$

On hydrogène 4 g du composé de l'EXEMPLE 3 en présence de 0,4 g de palladium sur charbon à 10% et de 50 ml d'éthanol. On filtre et évapore le filtrat sous pression réduite et purifie le résidu obtenu sur une colonne de gel de silice en éluant avec un mélange toluène/éthanol 97/3 (v/v). On reprend le résidu huileux obtenu avec du éther diéthylique, fait barboter de l'acide chlorhydrique. On filtre et sèche le précipité obtenu ; F = 154°C (HCl).

De la même manière on prépare les composés des EXEMPLES 69 à 78 présentés ci-après :

## TABLEAU 14

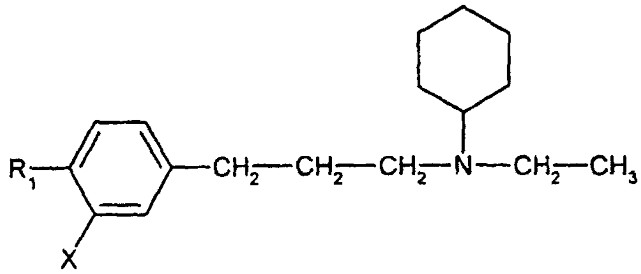

(I)

| EXEMPLE | $R_1$ | X | F ; °C (sel, hydrate) |
|---|---|---|---|
| 69 | 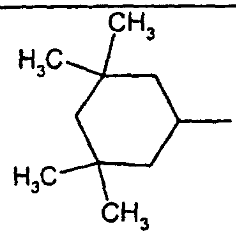 | H | 170<br>HCl<br>0,2 $H_2O$ |
| 70 | 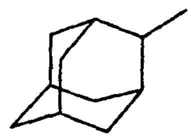 | H | 182<br>HCl<br>0,6 $H_2O$ |
| 71 | 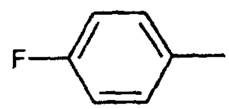 | Cl | 129<br>HCl |
| 72 | 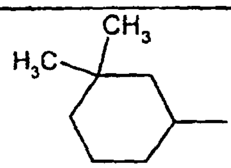 | Cl | 184<br>HCl |
| 73 | 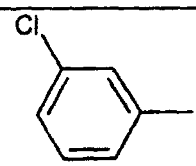 | Cl | 102<br>HCl<br>1,2 $H_2O$ |
| 74 | 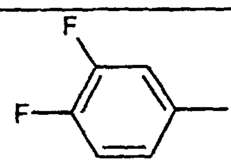 | Cl | 104<br>HCl |
| 75 | 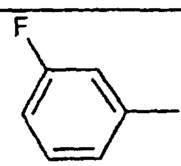 | Cl | 88<br>HCl<br>0,7 $H_2O$ |

**TABLEAU 14 (suite 1)**

| EXEMPLE | $R_1$ | X | F ; °C (sel, hydrate) |
|---|---|---|---|
| 76 | 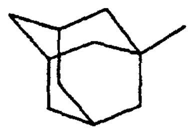 | H | 228<br>HCl |

**EXEMPLE 77**

**Chlorhydrate de [3-(2,6-dichlorophényl-4-yl)propyl]cyclohexyléthylamine**

(I) : $R_1$ = 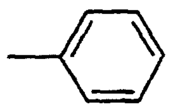 ; X = 3-Cl ; Y = 6-Cl ; A = $-CH_2-CH_2$

F = 128°C (HCl)

**EXEMPLE 78**

**Chlorhydrate de {-3-[4-(2-adamantyl)-3,5-dichlorophényl]propyl}cyclohexyl éthylamine**

(I) : $R_1$ = 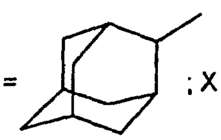 ; X = 3-Cl ; Y = 5-Cl ; A = $-CH_2-CH_2-$

F = 220°C (HCl)

**Revendications**

**1.** Composés de formule :

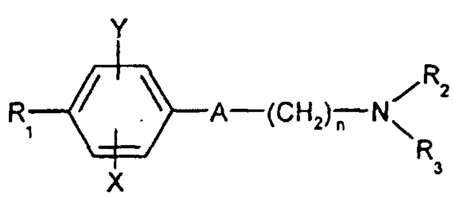

(I)

dans laquelle :

- A représente un groupe choisi parmi les suivants : $-C\equiv C-$ , $-CH=CH-$ ; $-CH_2-CH_2-$
- n est égal à 1 ou 2 ;
- X représente un atome d'hydrogène, de chlore ou de fluor ou un groupe méthyle ou méthoxy ;

- Y représente un atome d'hydrogène ou un atome de chlore ou de fluor ;
- $R_1$ représente un groupe cyclohexyle monosubstitué, disubstitué, trisubstitué ou tétrasubstitué par un groupe méthyle ; un groupe phényle monosubstitué ou disubstitué par un atome de fluor ou de chlore ou par un groupe méthoxy ; un groupe cycloheptyle, *tert*-butyle, dicyclopropylméthyle, bicyclo[3.2.1]octanyle, 4-tétrahydropyranyle, 4-tétrahydrothiopyranyle ou adamantyle 1 ou 2 ou adamantan-2-ol ; ou $R_1$ représente un groupe phényle étant entendu que dans ce cas X et Y sont différents de l'hydrogène ;
- $R_2$ représente un atome d'hydrogène ou un groupe ($C_1$-$C_4$)alkyle éventuellement substitué par un groupe trifluorométhyle;
- $R_3$ représente un ($C_5$-$C_7$)cycloalkyle ;

et les sels d'addition de ces composés avec des acides pharmaceutiquement acceptables, ainsi que leurs solvats et hydrates.

**2.** Composés selon la revendication 1 dans lesquels :

- A représente un groupe choisi parmi les suivants :
  $-C{\equiv}C-$ , -CH=CH- ; $-CH_2-CH_2-$
- n est égal à 1 ou 2 ;
- X représente un atome d'hydrogène, de chlore ou de fluor ou un groupe méthyle ou méthoxy ;
- Y représente un atome d'hydrogène ou un atome de chlore ou de fluor ;
- $R_1$ représente un groupe cyclohexyle monosubstitué, disubstitué, trisubstitué ou tétrasubstitué par un groupe méthyle ; un groupe phényle monosubstitué ou disubstitué par un atome de fluor ou de chlore ou par un groupe méthoxy ; un groupe cycloheptyle, *tert*-butyle, dicyclopropylméthyle, bicyclo[3.2.1]octanyle, 4-tétrahydropyranyle, 4-tétrahydrothiopyranyle ou adamantyle 1 ou 2 ; ou $R_1$ représente un groupe phényle étant entendu que dans ce cas X et Y sont différents de l'hydrogène ;
- $R_2$ représente un ($C_1$-$C_4$)alkyle éventuellement substitué par un groupe trifluorométhyle;
- $R_3$ représente un ($C_5$-$C_7$)cycloalkyle ;

et les sels d'addition de ces composés avec des acides pharmaceutiquement acceptables, ainsi que leurs solvats et hydrates.

**3.** Composés selon la revendication 1 ou 2 de formule :

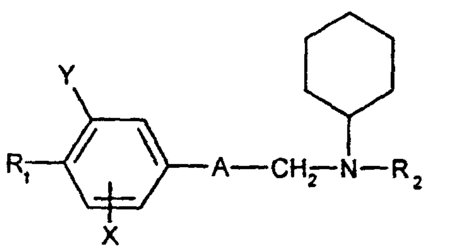

(I.1)

dans laquelle :

- A représente un groupe choisi parmi les suivants :
  $-C{\equiv}C-$ ; -CH=CH- ; $-CH_2-CH_2-$
- X représente un atome d'hydrogène ou de chlore ;
- Y représente un atome d'hydrogène ou un atome de chlore;
- $R_1$ représente un cyclohexyle monosubstitué, disubstitué, trisubstitué ou tétrasubstitué par un groupe méthyle ; un groupe phényle substitué par un atome de chlore, un groupe méthoxy ou un ou deux atomes de fluor ; un groupe *tert*-butyle ou adamantyle 1 ou 2 ; ou $R_1$ représente un groupe phényle étant entendu que dans ce cas X et Y représentent tous les deux un atome de chlore ;
- $R_2$ représente un ($C_2$-$C_3$)alkyle ;

et les sels d'addition de ces composés avec des acides pharmaceutiquement acceptables, ainsi que leurs solvats et hydrates.

**4.** Composés selon l'une quelconque des revendications 1 à 3 dans lesquels A représente le groupe -CH=CH- de configuration (Z).

**5.** Composés selon l'une quelconque des revendications 1 à 4 dans lesquels X représente un atome de chlore et Y représente un atome d'hydrogène ou de chlore.

**6.** Composés selon l'une quelconque des revendications 1 à 5 dans lesquels $R_1$ représente le groupe 3,3,5,5-tétraméthylcyclohexyle ou 3,3-diméthylcyclohexyle ou 4,4-diméthylcyclohexyle, un groupe phényle monosubstitué ou disubstitué par un atome de fluor ou substitué en position 4 par un atome de chlore ; ou un groupe adamantyle 1 ou 2.

**7.** Composés selon la revendication 1 choisis parmi :

- [(Z)-3-(4-Adamantan-2-yl-3-chlorophényl)propèn-2-yl]cyclohexyléthylamine ;
- [(Z)-3-(4-Adamantan-2-ylphényl)propèn-2-yl]cyclohexyléthylamine ;
- {(Z)-3-[4-(4,4-Diméthylcyclohexyl)-2-chlorophényl]propèn-2-yl}cyclohexyléthylamine ;
- [(Z)-3-(4-Adamantan-1-yl-3-chlorophényl)propèn-2-yl]cyclohexyléthylamine ;
- [(Z)-3-(4-Adamantan-2-yl-3,5-dichlorophényl)propèn-2-yl]cyclohexyléthylamine ;
- [(Z)-3-(4-Adamantan-2-yl-3,5-dichlorophényl)propèn-2-yl]cyclohexyl(2-méthyléthyl) amine ;

ainsi que leurs sels avec des acides pharmaceutiquement acceptables, leurs solvats et hydrates.

**8.** [(Z)-3-(4-Adamantan-2-yl-3,5-dichlorophényl) propèn-2-yl)cyclohexyléthylamine ainsi que ses sels avec des acides pharmaceutiquement acceptables, solvats et hydrates selon la revendication 7.

**9.** Procédé pour la préparation d'un composé selon la revendications 1 dans lequel A représente le groupement -C≡C- **caractérisé en ce que** :

a) soit, si n = 1, on effectue une réaction de Mannich entre le dérivé phénylacétylénique de formule :

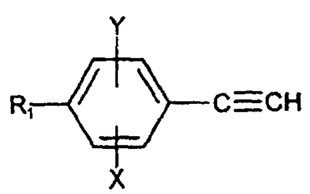

(II)

dans laquelle $R_1$, X et Y sont tels que définis pour (I), le formaldéhyde et l'amine (1) $HNR_2R_3$, $R_2$ et $R_3$ étant tels que définis pour (I) ;
b) soit on effectue un couplage de Suzuki entre le composé de formule :

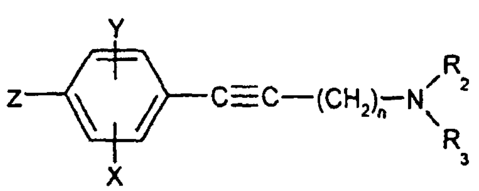

(Ia)

dans laquelle X, Y, n, $R_2$ et $R_3$ sont tels que définis pour (I) et Z représente un brome, un iode ou le groupe trifluorométhanesulfonate (OTf) et un dérivé boronique (2) de formule $R_1$-$B(OR)_2$ dans lequel $R_1$ est tel que défini pour (I) et R représente un atome d'hydrogène, un groupe alkyle ou aryle en présence d'une base et d'un catalyseur métallique ;
c) soit lorsque $R_1$ représente un groupe cyclohexyle monosubstitué, disubstitué, trisubstitué ou tétrasubstitué par un groupe méthyle ; un groupe cycloheptyle, 4-tétrahydropyranyle, 4-tétrahydrothiopyranyle ou adamantyle, on effectue un couplage entre le composé (Ia) dans lequel Z représente un atome d'iode ou de brome avec la cétone (3) correspondant à $R_1$ représentée par

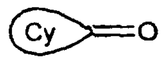

en présence d'une base pour obtenir intermédiairement le composé de formule :

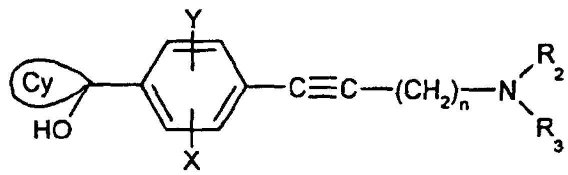

(I')

dans laquelle X, Y, n, $R_2$ et $R_3$ sont tels que définis pour (I) ; ledit composé (I') étant ensuite réduit dans des conditions sélectives ;

d) soit on effectue une réaction de couplage entre l'amine de formule :

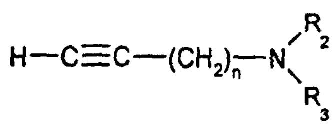

(4)

dans laquelle n, $R_2$ et $R_3$ sont tels que définis pour (I) et le composé de formule :

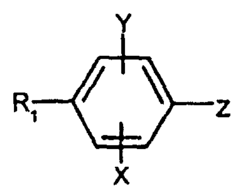

(III)

dans laquelle $R_1$, X et Y sont tels que définis pour (I) et Z représente un atome de brome, iode ou un groupe trifluorométhylsulfonate (triflate ou OTf).

**10.** Procédé pour la préparation d'un composé selon la revendication 1 dans lequel A représente le groupe -CH=CH- **caractérisé en ce qu'**on effectue une hydrogénation par l'hydrogène naissant ou en présence de cyclohexène du composé (I) dans lequel A représente le groupe acétylénique -C≡C- pour préparer le composé éthylénique (I) sous forme d'un mélange des isomères Z et E ou on effectue cette hydrogénation en présence d'un catalyseur métallique sur support pour préparer le composé éthylénique (I) sous forme Z, ou encore on fait réagir sur le composé (I) dans lequel A représente le groupe acétylénique -C≡C- un hydrure métallique pour préparer le composé éthylénique (I) sous forme E.

**11.** Procédé pour la préparation d'un composé selon la revendication 1 dans lequel A représente le groupe $-CH_2-CH_2-$ **caractérisé en ce qu'**on effectue une hydrogénation du composé (I) dans lequel A représente un groupe -CH=CH- ou -C≡C-.

**12.** Composition pharmaceutique contenant à titre de principe actif un composé selon l'une quelconque des revendications 1 à 8.

**13.** Utilisation d'un composé selon l'une quelconque des revendications 1 à 8 pour la préparation d'un médicament destiné à traiter les maladies où il est souhaitable de réduire l'activité immunologique, en particulier les maladies auto-immunes.

**14.** Utilisation d'un composé selon l'une quelconque des revendications 1 à 8 pour la préparation d'un médicament destiné à s'opposer à la prolifération des cellules tumorales.

**15.** Utilisation d'un composé selon l'une quelconque des revendications 1 à 8 pour la préparation d'un médicament destiné à traiter les troubles du rythme cardiaque.

## Patentansprüche

**1.** Verbindungen der Formel:

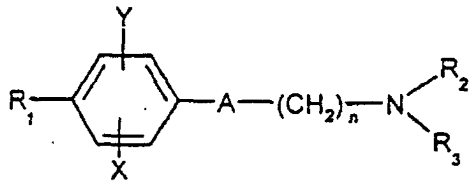

(I)

in der:

- A eine Gruppe ausgewählt aus:
  -C≡C- , -CH=CH- ; $-CH_2-CH_2-$ ;
- n 1 oder 2;
- X ein Wasserstoff-, Chlor- oder Fluor-Atom oder eine Methyl- oder MethoxyGruppe;
- Y ein Wasserstoff-, Chlor- oder Fluor-Atom,
- $R_1$ eine einfach, zweifach, dreifach oder vierfach durch eine Methylgruppe substituierte Cyclohexylgruppe; eine einfach oder zweifach durch ein Fluor- oder Chlor-Atom oder eine Methoxygruppe substituierte Phenylgruppe; eine Cycloheptylgruppe, tert.-Butylgruppe, Dicyclopropylmethylgruppe, Bicyclo[3.2.1]octanylgruppe, 4-Tetrahydropyranylgruppe, 4-Tetrahydrothiopyranylgruppe oder Adamantyl-1- oder Adamantyl-2-Gruppe oder eine Adamantan-2-ol-Gruppe; oder $R_1$ eine Phenylgruppe, wobei in diesem Fall X und Y von Wasserstoff verschieden sind;
- $R_2$ ein Wasserstoffatom oder eine gegebenenfalls durch eine Trifluormethylgruppe substituierte $(C_1-C_4)$-Alkylgruppe; und
- $R_3$ eine $(C_5-C_7)$-Cycloalkylgruppe bedeuten;

sowie die Additionssalze dieser Verbindungen mit pharmazeutisch annehmbaren Säuren sowie ihre Solvate und Hydrate.

**2.** Verbindung nach Anspruch 1, worin:

- A eine Gruppe ausgewählt aus:
  -C≡C- , -CH=CH- ; $-CH_2-CH_2-$ ;
- n 1 oder 2;
- X ein Wasserstoff-, Chlor- oder Fluor-Atom oder eine Methyl- oder MethoxyGruppe;
- Y ein Wasserstoffatom oder ein Chlor- oder Fluor-Atom,
- $R_1$ eine einfach, zweifach, dreifach oder vierfach mit einer Methylgruppe substituierte Cyclohexylgruppe; eine einfach oder zweifach durch ein Fluor- oder Chlor-Atom oder eine Methoxygruppe substituierte Phenylgruppe; eine Cycloheptylgruppe, tert.-Butylgruppe, Dicyclopropylmethylgruppe, Bicyclo[3.2.1]octanylgruppe, 4-Tetrahydropyranylgruppe, 4-Tetrahydrothlopyranylgruppe oder Adamantyl-1-oder-2-Gruppe; oder $R_1$ eine Phenylgruppe, wobei in diesem Fall X und Y von Wasserstoff verschieden sind;
- $R_2$ eine gegebenenfalls durch eine Trifluormethylgruppe substituierte $(C_1-C_4)$-Alkylgruppe; und
- $R_3$ eine $(C_5-C_7)$-Cycloalkylgruppe bedeuten;

und die Additionssalze dieser Verbindungen mit pharmazeutisch annehmbaren Säuren sowie ihre Solvate und Hydrate.

**3.** Verbindungen nach Anspruch 1 oder 2 der Formel:

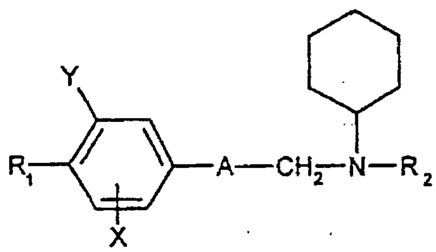

(I.1)

in der:

- A eine Gruppe ausgewählt aus:
  -C≡C- ; -CH=CH- ; $-CH_2-CH_2-$ ;
- X ein Wasserstoff- oder Chloratom;
- Y ein Wasserstoff- oder Chloratom;
- $R_1$ eine einfach, zweifach, dreifach oder vierfach durch eine Methylgruppe substituierte Cyclohexylgruppe; eine durch ein Chlor-Atom, eine Methoxygruppe oder ein oder zwei Fluoratome substituierte Phenylgruppe; eine tert.-Butyl- oder Adamantyl-1- oder -2-Gruppe; oder $R_1$ eine Phenylgruppe, wobei in diesem Fall X und Y jeweils Chloratome darstellen; und
- $R_2$ eine $(C_2-C_3)$-Alkylgruppe bedeuten;

und die Additionssalze dieser Verbindungen mit pharmazeutisch annehmbaren Säuren sowie ihre Solvate und Hydrate.

**4.** Verbindungen nach einem der Ansprüche 1 bis 3, worin A die Gruppe
-CH=CH- in der Konfiguration (Z) bedeutet.

**5.** Verbindungen nach einem der Ansprüche 1 bis 4, worin X ein Chloratom und Y ein Wasserstoffatom oder ein Chloratom bedeuten.

**6.** Verbindungen nach einem der Ansprüche 1 bis 5, worin $R_1$ die 3,3,5,5-Tetramethylcyclohexylgruppe oder die 3,3-Dimethylcyclohexylgruppe oder die 4,4-Dimethylcyclohexylgruppe, eine einfach oder zweifach durch ein Fluoratom oder in der 4-Stellung durch ein Chloratom substituierte Phenylgruppe oder eine Adamantyl-1- oder -2-Gruppe bedeutet.

**7.** Verbindungen nach Anspruch 1 ausgewählt aus:

- [(Z)-3-(4-Adamantan-2-yl-3-chlorphenyl)-propen-2-yl]-cyclohexylethylamin;
- [(Z)-3-(4-Adamantan-2-ylphenyl)-propen-2-yl]-cyclohexylethylamin;
- {(Z)-3-[4-(4,4-Dimethylcyclohexyl)-2-chlorphenyl]-propen-2-yl}-cyclohexylethylamin;
- [(Z)-3-(4-Adamantan-1-yl-3-chlorphenyl)-propen-2-yl]-cyclohexylethylamin;
- [(Z)-3-(4-Adamantan-2-yl-3,5-dichlorphenyl)-propen-2-yl]-cyclohexylethylamin;
- [(Z)-3-(4-Adamantan-2-yl-3,5-dichlorphenyl)-propen-2-yl]-cyclohexyl-(2-methylethyl)amin;

sowie deren Salze mit pharmazeutisch annehmbaren Säuren, deren Solvate und Hydrate.

**8.** [(Z)-3-(4-Adamantan-2-yl-3,5-dichlorphenyl)-propen-2-yl]-cyclohexylethylamin sowie dessen Salze mit pharmazeutisch annehmbaren Säuren, Solvate und Hydrate nach Anspruch 7.

**9.** Verfahren zur Herstellung einer Verbindung nach Anspruch 1, worin A die Gruppe -C≡C- darstellt, **dadurch gekennzeichnet, daß** man:

a) entweder wenn n = 1 bedeutet, eine Mannich-Reaktion zwischen dem Phenylacetylenderivat der Formel:

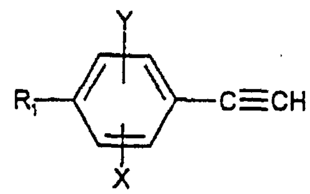

(II)

in der $R_1$, X und Y die bezüglich (I) angegebenen Bedeutungen besitzen; Formaldehyd und dem Amin (1) $HNR_2R_3$, worin $R_2$ und $R_3$ die bezüglich (I) angegebenen Bedeutungen besitzen, durchführt;
b) oder eine Suzuki-Kupplung zwischen der Verbindung der Formel:

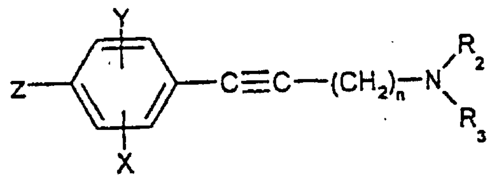

(Ia)

in der X, Y, n, $R_2$ und $R_3$ die bezüglich (I) angegebenen Bedeutungen besitzen und Z Brom, Iod oder die Trifluormethansulfonat-Gruppe (OTf) bedeutet und einem Boronderivat (2) der Formel $R_1$-$B(OR)_2$, worin $R_1$ die für (I) angegebenen Bedeutungen besitzt und R ein Wasserstoffatom, eine Alkylgruppe oder eine Arylgruppe darstellt, in Gegenwart einer Base und eines Metallkatalysators durchführt;
c) oder wenn $R_1$ eine einfach, zweifach, dreifach oder vierfach durch eine Methylgruppe substituierte Cyclohexylgruppe; eine Cycloheptylgruppe, 4-Tetrahydropyranylgruppe, 4-Tetrahydrothiopyranylgruppe oder Adamantylgruppe bedeutet, man eine Kupplung zwischen der Verbindung (Ia), in der Z ein Iod- oder Brom-Atom bedeutet, mit dem $R_1$ entsprechenden Keton (3) der Formel

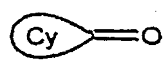

in Gegenwart einer Base durchführt, so daß man als Zwischenprodukt die Verbindung der Formel erhält:

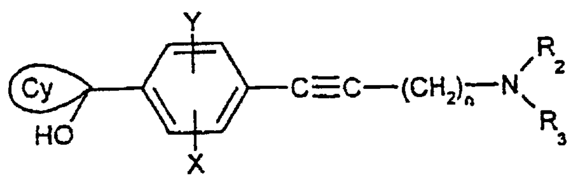

(I')

in der X, Y, n, $R_2$ und $R_3$ die für (I) angegebenen Bedeutungen besitzen; welche Verbindung (I') anschließend unter selektiven Bedingungen reduziert wird;
d) oder eine Kupplungsreaktion zwischen dem Amin der Formel:

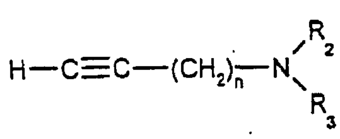

(4)

in der n, $R_2$ und $R_3$ die bezüglich (I) angegebenen Bedeutungen besitzen, und der Verbindung der Formel:

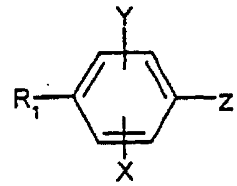

(III)

in der $R_1$, X und Y die für (I) angegebenen Bedeutungen besitzen und Z ein Bromatom, Iodatom oder eine Trifluormethylsulfonat-Gruppe (Triflat- oder OTf-Gruppe) darstellt, durchführt.

**10.** Verfahren zur Herstellung einer Verbindung nach Anspruch 1, worin A die Gruppe -CH=CH- darstellt, **dadurch gekennzeichnet, daß** man die Verbindung (I), in der A die Acetylengruppe -C≡C- darstellt, mit Hilfe von nascierendem Wasserstoff oder in Gegenwart von Cyclohexen reduziert zur Bildung der Ethylenverbindung (I) in Form einer Mischung der Isomeren Z und E oder diese Hydrierung in Gegenwart eines auf einem Träger vorliegenden Metallkatalysators durchführt zur Bildung der Ethylenverbindung (I) in der Form Z oder man die Verbindung (I), in der A die Acetylengruppe -C≡C- darstellt, mit einem Metallhydrid umsetzt zur Bildung der Ethylenverbindung (I) in der Form E.

**11.** Verfahren zur Herstellung einer Verbindung nach Anspruch 1, worin A die Gruppe $-CH_2-CH_2-$ darstellt, **dadurch gekennzeichnet, daß** man die Verbindung (I), in der A eine Gruppe -CH=CH- oder -C≡C- bedeutet, hydriert.

**12.** Pharmazeutische Zubereitung enthaltend als Wirkstoff eine Verbindung nach einem der Ansprüche 1 bis 8.

**13.** Verwendung einer Verbindung nach einem der Ansprüche 1 bis 8 für die Herstellung eines Arzneimittels zur Behandlung von Erkrankungen, bei denen es erwünscht ist, die immunologische Aktivität zu verringern, insbesondere bei Autoimmunerkrankungen.

**14.** Verwendung einer Verbindung nach einem der Ansprüche 1 bis 8 für die Herstellung eines Arzneimittels zur Bekämpfung der Vermehrung von Tumorzellen.

**15.** Verwendung einer Verbindung nach einem der Ansprüche 1 bis 8 für die Herstellung eines Arzneimittels zur Behandlung von Herzrhythmusstörungen.

## Claims

**1.** Compounds of formula:

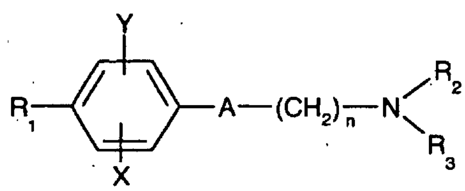

(I)

in which:

- A represents a group chosen from the following:
  -C≡C- , -CH=CH- ; $-CH_2-CH_2-$
- n is equal to 1 or 2;
- X represents a hydrogen, chlorine or fluorine atom or a methyl or methoxy group;
- Y represents a hydrogen atom or a chlorine or fluorine atom;
- $R_1$ represents a cyclohexyl group monosubstituted, disubstituted, trisubstituted or tetrasubstituted with a methyl group; a phenyl group monosubstituted or disubstituted with a fluorine or chlorine atom or with a methoxy group; a cycloheptyl, *tert*-butyl, dicyclopropylmethyl, bicyclo[3.2.1]octanyl, 4-tetrahydropyranyl, 4-tetrahydrothiopyranyl or 1- or 2-adamantyl or adamantan-2-ol group; or $R_1$ represents a phenyl group, it being understood that, in this case, X and Y are other than hydrogen;

- $R_2$ represents a hydrogen atom or a ($C_1$-$C_4$)alkyl group optionally substituted with a trifluoromethyl group;
- $R_3$ represents a ($C_5$-$C_7$)cycloalkyl;

and the addition salts of these compounds with pharmaceutically acceptable acids, as well as the solvates and hydrates thereof.

**2.** Compounds according to Claim 1, in which:

- A represents a group chosen from the following: $-C{\equiv}C-$ , $-CH{=}CH-$ ; $-CH_2-CH_2-$
- n is equal to 1 or 2;
- X represents a hydrogen, chlorine or fluorine atom or a methyl or methoxy group;
- Y represents a hydrogen atom or a chlorine or fluorine atom;
- $R_1$ represents a cyclohexyl group monosubstituted, disubstituted, trisubstituted or tetrasubstituted with a methyl group; a phenyl group monosubstituted or disubstituted with a fluorine or chlorine atom or with a methoxy group; a cycloheptyl, *tert*-butyl, dicyclopropylmethyl, bicyclo[3.2.1]octanyl, 4-tetrahydropyranyl, 4-tetrahydrothiopyranyl or 1- or 2-adamantyl group; or $R_1$ represents a phenyl group, it being understood that; in this case, X and Y are other than hydrogen;
- $R_2$ represents a ($C_1$-$C_4$)alkyl optionally substituted with a trifluoromethyl group;
- $R_3$ represents a ($C_5$-$C_7$)cycloalkyl;

and the addition salts of these compounds with pharmaceutically acceptable acids, as well as the solvates and hydrates thereof.

**3.** Compounds according to Claim 1 or 2, of formula:

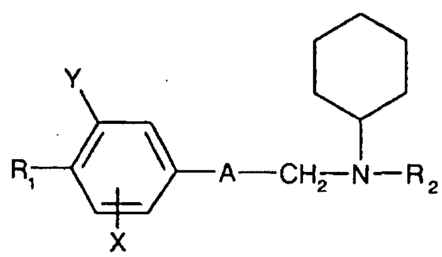

(I.1)

in which:

A represents a group chosen from the following:
$-C{\equiv}C-$ , $-CH{=}CH-$ ; $-CH_2-CH_2-$
- X represents a hydrogen or chlorine atom;
- Y represents a hydrogen atom or a chlorine atom;
- $R_1$ represents a cyclohexyl monosubstituted, disubstituted, trisubstituted or tetrasubstituted with a methyl group; a phenyl group substituted with a chlorine atom, a methoxy group or one or two fluorine atoms; a *tert*-butyl or 1- or 2-adamantyl group; or $R_1$ represents a phenyl group, it being understood that, in this case, X and Y both represent a chlorine atom;
- $R_2$ represents a ($C_2$-$C_3$)alkyl;

and the addition salts of these compounds with pharmaceutically acceptable acids, as well as the solvates and hydrates thereof.

**4.** Compounds according to any of Claims 1 to 3, in which A represents a -CH=CH- group of (Z) configuration.

**5.** Compounds according to any one of Claims 1 to 4, in which X represents a chlorine atom and Y represents a hydrogen or chlorine atom.

**6.** Compounds according to any one of Claims 1 to 5, in which $R_1$ represents a 3,3,5,5-tetramethylcyclohexyl or 3,3-dimethylcyclohexyl or 4,4-dimethylcyclohexyl group, a phenyl group monosubstituted or disubstituted with a fluorine atom or substituted in position 4 with a chlorine atom; or a 1- or 2-adamantyl group.

**7.** Compounds according to Claim 1, chosen from:

- [(Z)-3-(4-Adamantan-2-yl-3-chlorophenyl)propen-2-yl]cyclohexylethylamine;
- [(Z)-3-(4-Adamantan-2-ylphenyl)propen-2-yl]cyclohexylethylamine;
- {(Z)-3-[4-(4,4-Dimethylcyclohexyl)-2-chlorophenyl]propen-2-yl}cyclohexylethylamine;
- [(Z)-3-(4-Adamantan-l-yl-3-chlorophenyl)propen-2-yl]cyclohexylethylamine;
- [(Z)-3-(4-Adamantan-2-yl-3,5-dichlorophenyl)propen-2-yl]cyclohexylethylamine;
- [(Z)-3-(4-Adamantan-2-yl-3,5-dichlorophenyl)propen-2-yl]cyclohexyl(2-methylethyl)amine;

as well as the salts thereof with pharmaceutically acceptable acids and the solvates and hydrates thereof.

**8.** [(Z)-3-(4-Adamantan-2-yl-3,5-dichlorophenyl)propen-2-yl]cyclohexylethylamine as well as the salts thereof with pharmaceutically acceptable acids, solvates thereof and hydrates thereof according to Claim 7.

**9.** Process for preparing a compound according to Claim 1, in which A represents a -C≡C- group, **characterized in that**:

a) either, if n = 1, a Mannich reaction is carried out between the phenylacetylene derivative of formula :

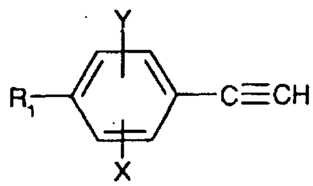

(II)

in which $R_1$, X and Y are as defined for (I), the formaldehyde and the amine (1) $HNR_2R_3$, $R_2$ and $R_3$ being as defined for (I);
b) or, a Suzuki coupling is carried out between the compound of formula:

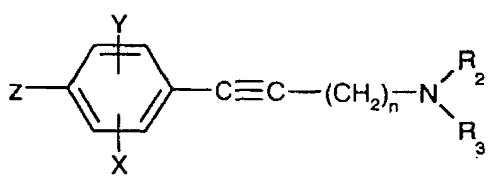

(Ia)

in which X, Y, n, $R_2$ and $R_3$ are as defined for (I) and Z represents a bromine, an iodine or a trifluoromethanesulphonate (OTf) group and a boron derivative (2) of formula $R_1$-B(OR)$_2$ in which $R_1$ is as defined for (I) and R represents a hydrogen atom or an alkyl or aryl group in the presence of a base and a metal catalyst;
c) or, when $R_1$ represents a cyclohexyl group monosubstituted, disubstituted, trisubstituted or tetrasubstituted with a methyl group; a cycloheptyl, 4-tetrahydropyranyl, 4-tetrahydrothiopyranyl or adamantyl group, a coupling is carried out between compound (Ia) in which Z represents an iodine or bromine atom and the ketone (3) corresponding to $R_1$ represented by

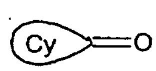

in the presence of a base, to give the intermediate compound of formula:

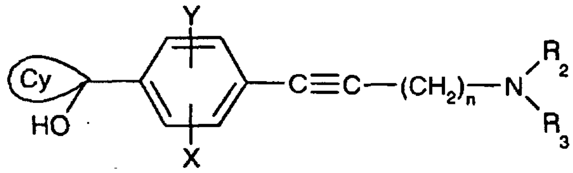 (I')

in which X, Y, n, $R_2$ and $R_3$ are as defined for (I) ; the said compound (I') then being reduced under selective conditions;

d) or, a coupling reaction is carried out between the amine of formula:

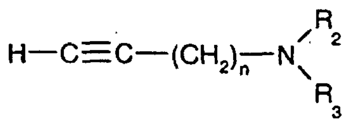 (4)

in which n, $R_2$ and $R_3$ are as defined for (I), and the compound of formula :

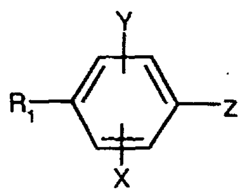 (III)

in which $R_1$, X and Y are as defined for (I) and Z represents a bromine or iodine atom or a trifluoromethylsulphonate (triflate or OTf) group.

**10.** Process for preparing a compound according to Claim 1, in which A represents a -CH=CH- group, **characterized in that** a hydrogenation is carried out, with nascent hydrogen or in the presence of cyclohexene, of compound (I) in which A represents an acetylene group -C≡C-, in order to prepare the ethylenic compound (I) in the form of a mixture of the Z and E isomers, or this hydrogenation is carried out in the presence of a metal catalyst on a support in order to prepare the ethylenic compound (I) in Z form, or alternatively compound (I) in which A represents an acetylene group -C≡C- is reacted with a metal hydride in order to prepare the ethylenic compound (I) in E form.

**11.** Process for preparing a compound according to Claim 1, in which A represents a $-CH_2-CH_2-$group **characterized in that** a hydrogenation of compound (I), in which A represents a -CH=CH- or -C≡C- group, is carried out.

**12.** Pharmaceutical composition containing, as active principle, a compound according to any one of Claims 1 to 8.

**13.** Use of a compound according to any one of Claims 1 to 8, for the preparation of a medicinal product intended for treating conditions in which it is desirable to reduce the immunological activity, in particular autoimmune conditions.

**14.** Use of a compound according to any one of Claims 1 to 8, for the preparation of a medicinal product intended for combating the proliferation of tumour cells.

**15.** Use of a compound according to any one of Claims 1 to 8, for the preparation of a medicinal product intended for treating heart rate disorders.